(19) **Europäisches Patentamt**
**European Patent Office**
**Office européen des brevets**

(11) **EP 3 274 885 B1**

(12) **EUROPEAN PATENT SPECIFICATION**

(45) Date of publication and mention
of the grant of the patent:
**07.07.2021 Bulletin 2021/27**

(51) Int Cl.:
*G16H 50/20* (2018.01)     *G16B 40/30* (2019.01)

(21) Application number: **16712848.7**

(86) International application number:
**PCT/EP2016/056670**

(22) Date of filing: **24.03.2016**

(87) International publication number:
**WO 2016/156251 (06.10.2016 Gazette 2016/40)**

(54) **METHOD FOR DETERMINING GASTROINTESTINAL TRACT DYSBIOSIS**

VERFAHREN ZUR BESTIMMUNG VON VERDAUUNGSTRAKT-DYSBIOSE

PROCÉDÉ DE DÉTERMINATION D'UNE DYSBIOSE DU TRACTUS GASTRO-INTESTINAL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **27.03.2015 GB 201505364**

(43) Date of publication of application:
**31.01.2018 Bulletin 2018/05**

(73) Proprietor: **Genetic Analysis AS**
**0580 Oslo (NO)**

(72) Inventors:
• **LINDAHL, Torbjørn**
**0510 Oslo (NO)**
• **KARLSSON, Magdalena**
**2405 Langhus (NO)**
• **SEKELJA, Monika**
**0379 Oslo (NO)**
• **HEGGE, Finn**
**0510 Oslo (NO)**

(74) Representative: **Dehns**
**St. Bride's House**
**10 Salisbury Square**
**London EC4Y 8JD (GB)**

(56) References cited:
**WO-A1-2014/022441**

• **CLARISSA SCHWAB ET AL: "Longitudinal study
of murine microbiota activity and interactions
with the host during acute inflammation and
recovery", THE I S M E JOURNAL:
MULTIDISCIPLINARY JOURNAL OF MICROBIAL
ECOLOGY, vol. 8, no. 5, 1 May 2014 (2014-05-01),
pages 1101-1114, XP055278936, United Kingdom
ISSN: 1751-7362, DOI: 10.1038/ismej.2013.223**
• **IAN H MCHARDY ET AL: "Integrative analysis of
the microbiome and metabolome of the human
intestinal mucosal surface reveals exquisite
inter-relationships", MICROBIOME, BIOMED
CENTRAL LTD, LONDON, UK, vol. 1, no. 1, 5 June
2013 (2013-06-05), page 17, XP021154335, ISSN:
2049-2618, DOI: 10.1186/2049-2618-1-17**
• **Anonymous: "Principal component analysis -
Wikipedia, the free encyclopedia", Wikipedia, 17
March 2013 (2013-03-17), pages 1-12,
XP055117197, Retrieved from the Internet:
URL:http://web.archive.org/web/20130317012
102/http://en.wikipedia.org/wiki/Principal
_component_analysis [retrieved on 2014-05-09]**
• **Anonymous: "Talk:Principal component
analysis - Wikipedia, the free encyclopedia", , 21
June 2013 (2013-06-21), XP055278969, Retrieved
from the Internet:
URL:https://en.wikipedia.org/wiki/Talk:Pri
ncipal_component_analysis [retrieved on
2016-06-08]**
• **Anonymous: "T-Squared Q residuals and
Contributions - Eigenvector Documentation
Wiki", , 28 June 2012 (2012-06-28), XP055278858,
Retrieved from the Internet:
URL:http://wiki.eigenvector.com/index.php?
title=T-Squared_Q_residuals_and_Contributi
ons [retrieved on 2016-06-08]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention provides means by which the state of the microbiota of the GI tract may be assessed and deviations from the normal state (normobiosis), i.e. dysbiosis, may be determined in a manner which is straightforward to perform, reliable and robust and which is flexible enough to be used with any technique for measuring levels of microorganisms in a GI tract sample. Such information may be used in the diagnosis, monitoring and/or characterisation of diseases and conditions associated with perturbations in the microbiota of the gastrointestinal (GI) tract. In more specific embodiments such deviations may be to an extent quantified and thus the invention provides the means of determining the extent of GI tract dysbiosis, which may in turn indicate the severity of the associated disease or condition or may be used to monitor the progression of or characterise the associated disease or condition.

The GI tract, also referred to as the digestive tract or alimentary canal (and which terms may be used interchangeably with GI tract), is the continuous series of organs beginning at the mouth and ending at the anus. Throughout its length the GI tract is colonised by microorganisms of a variety of different species. Together the microorganism content of the GI tract is the microbiota of the GI tract. The relative amounts of the constituent microorganisms or groups thereof can be considered to be a profile of the microbiota. Microbiota profiles therefore give information on microbial diversity (i.e. the number of taxonomically distinct microbes or distinct taxonomic groups which are present) in the GI tract as well as providing information on the relative amounts of the microbes or groups thereof which are present.

Many diseases and conditions, or stages thereof, are believed to be associated with perturbations in the microbiota of the GI tract, or regions thereof. In some instances the disease or condition may be caused by, or is exacerbated by, the shift in the profile of the microbiota of the GI tract, or regions thereof (i.e. the relative amounts of constituent microbes and the diversity of those microbes). In other instances the disease or condition causes, or by some mechanism results in, the display of a profile of the microbiota of the GI tract that differs from the normal state. In some contexts this may even be an adaptive response attempting to address the pathological phenotype of the disease or condition. Accordingly, by assessing the state of the microbiota of the GI tract and determining deviations from the normal state (normobiosis), i.e. dysbiosis, information can be provided that permits the diagnosis, monitoring and/or characterisation of diseases and conditions associated with perturbations in the microbiota of the gastrointestinal (GI) tract or that permits, or at least is useful in, an assessment of the risk of developing a disease or condition which has been determined to be associated by a perturbation of the microbiota profile.

Diseases and conditions affecting the GI tract are very likely to result in microbiota profiles that vary from the normal state, e.g. Inflammatory Bowel Disease (IBD), Crohn's Disease (CD), Ulcerative Colitis (UC), Irritable Bowel Syndrome (IBS), small bowel bacterial overgrowth syndrome and GI tract cancers (e.g. cancer of the mouth, pharynx, oesophagus, stomach, duodenum, jejunum, ileum, cecum, colon, rectum or anus) and evidence also exists of links between GI tract microbiota profiles and diseases and conditions that are considered to be unrelated to the GI tract, for instance breast cancer; ankylosing spondylitis; non-alcoholic steatohepatitis; the atopic diseases, e.g. eczema, asthma, atopic dermatitis, allergic conjunctivitis, allergic rhinitis and food allergies; metabolic disorders, e.g. diabetes mellitus (type 1 and type 2), obesity and metabolic syndrome; neurological disorders, e.g. depression, multiple sclerosis, dementia, and Alzheimer's disease; autoimmune disease (e.g. arthritis); malnutrition; chronic fatigue syndrome and autism. It is believed that such perturbations of the GI tract microbiota profile (in terms of relative amounts and/or diversity), which may be considered to equate to an imbalance in the GI tract microbiota, contribute to these diseases, either by causing the diseases or contributing to their progression. It is also believed that many more diseases will be found to have causal links to perturbations of the GI tract microbiota profile. The precise mechanism behind this causation is not well understood. It is clear that perturbation of the microbiota of the GI tract results in the underpopulation of certain microbes and/or the overpopulation of others and/or reductions in diversity and this causes a shift, or imbalance, in the relative activities of each microbe population. It is believed that this shift in microbial activities causes a reduction in beneficial effects (e.g. synthesis of vitamins, short-chain fatty acids and polyamines, nutrient absorption, inhibition of pathogens, metabolism of plant compounds) to occur and/or an increase in deleterious effects (secretion of endotoxins and other toxic products) to occur with consequent overall negative effects on the host's overall physiology. These effects can then manifest as illness and disease, e.g. those recited above.

Although it is now common place to determine and even quantify the relative amounts of microorganisms in a GI tract sample and to use such profiles to diagnose disease by reference to specific profiles characteristic of a disease state or to rule out a diagnosis by reference to specific profiles characteristic of a normal state (e.g. WO2012080754; WO2011043654) there remains a need for methods which determine the likelihood that a patient has GI tract dysbiosis, *vis a vis* a normobiotic state, which are straightforward to perform, reliable and robust and which are flexible enough to be used with any technique for measuring levels of microorganisms in a GI tract sample and which do not require reference to specific standard profiles.

Thus in a first aspect the invention provides a computer-implemented method for determining the likelihood of GI tract dysbiosis in a subject, said method comprising:

(i) providing a test data set, wherein said test data set comprises at least one microbiota profile, said microbiota profile being a profile of the relative levels of a plurality of microorganisms or groups of microorganisms in a sample from the GI tract of the subject and wherein each level of each microorganism or group of microorganisms is a profile element of said test data set,

(ii) applying to said test data set at least one loading vector, wherein said at least one loading vector is at least one orthogonal latent variable determined by the regression analysis of the levels of said plurality of microorganisms or groups of microorganisms in corresponding GI tract samples from a plurality of normal subjects, thereby producing a first projected data set,

(iii) providing said first projected data set,

(iv) from said first projected data set calculating the Q-residual of the microbiota profile and comparing the Q-residual of the microbiota profile with a normobiotic to dysbiotic threshold Q-residual value determined from the corresponding analysis of said plurality of microorganisms or groups of microorganisms in corresponding GI tract samples from a plurality of normal subjects and/or subjects with dysbiosis,

(v) from said first projected data set calculating the Hotelling's $T^2$ for the microbiota profile from the variance explained by the orthogonal latent variables determined by the regression analysis of the levels of said plurality of microorganisms or groups of microorganisms in corresponding GI tract samples from a plurality of normal subjects of step (ii) and comparing said Hotelling's $T^2$ for the microbiota profile with a normobiotic to dysbiotic threshold Hotelling's $T^2$ value determined from the corresponding analysis of said plurality of microorganisms or groups of microorganisms in corresponding GI tract samples from a plurality of normal subjects and/or a plurality of subjects with dysbiosis,

wherein step (v) may be performed before or after or concurrently with step (iv), and wherein a microbiota profile with a Q-residual or Hotelling's $T^2$ in excess of said respective thresholds is indicative of a likelihood of dysbiosis.

In other embodiments a likelihood of dysbiosis is indicated if both said Q-residual and said Hotelling's $T^2$ are in excess of their respective normobiotic to dysbiotic thresholds.

The method of the invention may also be considered to be a method to identify dysbiosis, to detect dysbiosis, to determine the presence of dysbiosis or characterise dysbiosis. The method of the invention may therefore be considered to comprise a step of determining the likelihood of GI tract dysbiosis, identifying dysbiosis, detecting dysbiosis, determining the presence of dysbiosis or characterising dysbiosis in the subject based on the indication of a likelihood of dysbiosis provided in the preceding steps. The results of such a step may be recorded and optionally stored on a suitable recording/storage medium and/or communicated to a physician, the subject or intermediary or agent thereof.

In certain embodiments the method is performed on a plurality of microbiota profiles. These profiles may be from the same subject, e.g. as parallel profiles obtained from the same sample, from different corresponding samples from the same subject, e.g. obtained from the subject at different times, or from different samples from the said subject. Alternatively, or in addition, corresponding samples from another subject may be analysed together with samples from the first subject. In these embodiments it may be convenient to arrange said microbiota profiles in a test data matrix, wherein each distinct profile element is aligned across the plurality of microbiota profiles.

In a particular example of these embodiments there is provided a computer-implemented method for determining the likelihood of GI tract dysbiosis in a subject, said method comprising:

(i) providing a plurality of microbiota profiles, wherein each of said microbiota profiles is a profile of the relative levels of a plurality of microorganisms or groups of microorganisms in

(a) a sample from the GI tract of the subject, or

(b) different corresponding samples from the GI tract of said subject, wherein each microbiota profile has been prepared in essentially the same way and wherein each level of each microorganism or group of microorganisms is a profile element, and arranging said microbiota profiles in a test data matrix, wherein each distinct profile element is aligned across the plurality of microbiota profiles,

(ii) applying to said test data matrix at least one loading vector wherein said at least one loading vector is at least one orthogonal latent variable determined by the regression analysis of the levels of said plurality of microorganisms or groups of microorganisms in corresponding GI tract samples from a plurality of normal subjects, thereby producing a first projected data matrix,

(iii) providing said first projected data matrix,

(iv) from said first projected data matrix calculating the Q-residual for each microbiota profile and comparing the Q-residual for each microbiota profile with a normobiotic to dysbiotic threshold Q-residual value determined from the corresponding analysis of said plurality of microorganisms or groups of microorganisms in corresponding GI tract samples from a plurality of normal subjects and/or subjects with dysbiosis,

(v) from said first projected data matrix calculating the Hotelling's $T^2$ for each microbiota profile from the variance explained by the orthogonal latent variables determined by the regression analysis of the levels of said plurality of microorganisms or groups of microorganisms in corresponding GI tract samples from a plurality of normal subjects of step (ii) and comparing said Hotelling's $T^2$ for each microbiota profile with a normobiotic to dysbiotic threshold Hotelling's $T^2$ value determined from the corresponding analysis of said plurality of microorganisms or groups of microorganisms in corresponding GI tract samples from a plurality of normal subjects and/or a plurality of subjects with dysbiosis,

wherein step (v) may be performed before or after or concurrently with step (iv), and wherein a microbiota profile with a Q-residual or Hotelling's $T^2$ in excess of said respective thresholds is indicative of a likelihood of dysbiosis in the GI tract of the subject from which it has been obtained.

In another particular example of these embodiments there is provided a method for determining the likelihood of GI tract dysbiosis in a plurality of subjects, said method comprising:

(i) providing a plurality of microbiota profiles, wherein each of said microbiota profiles is a profile of the relative levels of a plurality of microorganisms or groups of microorganisms in corresponding samples from the GI tract of said subjects which have been prepared in essentially the same way and wherein each level of each microorganism or group of microorganisms is a profile element, and arranging said microbiota profiles in a test data matrix, wherein each distinct profile element is aligned across the plurality of microbiota profiles,

and performing steps (ii) to (v) of the method of the invention described *supra,* wherein step (v) may be performed before or after or concurrently with step (iv), and wherein a microbiota profile with a Q-residual or Hotelling's $T^2$ in excess of said respective thresholds is indicative of a likelihood of dysbiosis in the GI tract of the subject from which it has been obtained.

The following sections describe the detail of the method of the invention in terms of the analysis of a single microbiota profile. These details apply *mutatis mutandis* to the above-described embodiments in which a plurality of microbiota profile are analysed together.

Expressed differently the method of the invention may be considered a method for detecting, diagnosing or monitoring GI tract dysbiosis in a subject wherein a microbiota profile with a Q-residual or Hotelling's $T^2$, typically both, in excess of said respective normobiotic to dysbiotic thresholds indicates dysbiosis.

Dysbiosis is defined herein as a microbiota profile that differs or deviates from the microbiota profile that is typical of a normal, healthy, subject, which may be referred to herein as "normobiosis" or a "normobiotic state". The extent of dysbiosis is a measure of how different a microbiota profile is from a normal microbiota profile or by how much a microbiota profile deviates from a normal microbiota profile. In the context of the diagnosis, monitoring and/or characterisation of diseases and conditions associated with perturbations in the microbiota of the GI tract or the assessment of the risk of developing a disease or condition which has been determined to be associated with a perturbation of the microbiota profile, dysbiosis may be more specifically defined as a microbiota profile that differs from the microbiota profile that is typical of a subject which does not have said disease or condition or is not at risk of developing said disease or condition. A typical microbiota profile may be obtained from a single subject or even a single sample from a single subject, but preferably will be obtained from a plurality of subjects.

A microbiota profile (profile of the relative levels of a plurality of microorganisms or groups of microorganisms) in accordance with the methods of the invention is a numerical representation of such levels that has been obtained from an analysis of a GI tract sample from the subject. The individual values for such levels (the individual profile elements) may be qualitative, quantitative or semi-quantitative, preferably quantitative. The term "amount" could be used in place of "levels" if appropriate.

The profiling of the GI tract sample may involve any convenient means by which the levels of microorganism or group of microorganisms may be measured, preferably quantified. Preferably the means used to prepare the microbiota profiles from a plurality of normal subjects and/or a plurality of subjects with dysbiosis from which the latent variables, e.g. orthogonal latent variables, of step (ii) and the threshold values of steps (iv) and (v) are determined are essentially the same as those used to prepare the at least one microbiota profile of the test data set, although in other embodiments the means may differ. Should different means be used an adjustment vector may need to be calculated and applied in order to permit comparison.

The profiling methods of use in accordance the invention are typically *in vitro* methods performed using any sample taken from the GI tract.

The GI tract, also referred to as the digestive tract or alimentary canal (and which terms may be used interchangeably with GI tract) is the continuous series of organs beginning at the mouth and ending at the anus. Specifically this sequence consists of the mouth, the pharynx, the oesophagus, the stomach, the duodenum, the small intestine, the large intestine and the anus. These organs can be subdivided into the upper GI tract, consisting of the mouth, pharynx, oesophagus, stomach, and duodenum, and the lower GI tract, consisting of the jejunum, the ileum (together the small intestine), the cecum, the colon, the rectum (together the large intestine) and the anus.

A GI tract sample of use in accordance with the invention may include, but is not limited to any fluid or solid taken from the lumen or surface of the GI tract or any sample of any of the tissues that form the organs of the GI tract. Thus the sample may be any luminal content of the GI tract (e.g. stomach contents, intestinal contents, mucus and faeces/stool, or combinations thereof) as well as samples obtained mechanically from the GI tract e.g. by swab, rinse, aspirate or scrape of a GI tract cavity or surface or by biopsy of a GI tract tissue/organ. Faecal samples are preferred. The sample can also be obtained from part of a GI tract tissue/organ which has been removed surgically. The sample may be a portion of the excised tissue/organ. In embodiments where the sample is a sample of a GI tract tissue/organ the sample may comprise a part of the mucosa, the submucosa, the muscularis externa, the adventitia and/or the serosa of the GI tract tissue/organ. Such tissue samples may be obtained by biopsy during an endoscopic procedure. Preferably the sample is obtained from the lower GI tract, i.e. from the jejunum, the ileum, the cecum, the colon, the rectum or the anus. More preferably the sample is a mucosal or luminal sample. Faecal samples may be collected by the swab, rinse, aspirate or scrape of the rectum or anus or, most simply, the collection of faeces during or after defecation.

The sample may be used in accordance with the invention in the form in which it was initially retrieved. The sample may also have undergone some degree of manipulation, refinement or purification before being used in the methods of the invention. Thus the term "sample" also includes preparations thereof, e.g. relatively pure or partially purified starting materials, such as semi-pure preparations of the above mentioned samples. The term "sample" also includes preparations of the above mentioned samples in which the RNA of which, including the 16S rRNA, has undergone reverse transcription. Further included is the product of the microbial culture of said sample.

The purification may be slight, for instance amounting to no more than the concentration of the solids, or cells, of the sample into a smaller volume or the separation of cells from some or all of the remainder of the sample. Representative cell isolation techniques are described in WO98/51693 and WO01/53525.

In certain embodiments a preparation of the nucleic acid from the above mentioned samples, preferably a preparation in which the nucleic acids have been labelled, is used in accordance with the invention. Such preparations include reverse transcription products and/or amplification products of such samples or nucleic acid preparations thereof. It may be advantageous if the predominant nucleic acid of the nucleic acid preparation is DNA. These preparations include relatively pure or partially purified nucleic acid preparations.

Techniques for the isolation of nucleic acid from samples, including complex samples, are numerous and well known in the art and described at length in the literature. The techniques described in WO98/51693 and WO01/53525 can also be employed to prepare nucleic acids from the above mentioned samples.

The method of the invention may include a step of sample collection and/or sample processing and/or culture, in particular a step of nucleic acid amplification, e.g. genomic nucleic acid amplification, in particular the amplification of nucleic acid carrying nucleotide sequences characteristic of a microorganism or group of microorganisms.

Unless context dictates otherwise, the term "corresponding sample" is used herein to refer to samples of the same type which have been obtained from different subjects and/or at different times in essentially the same way and to which any substantive processing or handling thereof has taken place in essentially the same way.

Methods for profiling microbiota in a GI tract sample include but are not limited to nucleic acid analysis (e.g. nucleic acid sequencing approaches, oligonucleotide hybridisation probe based approaches, primer based nucleic acid amplification approaches), antibody or other specific affinity ligand based approaches, proteomic and metabolomic approaches. Preferably the analysis of the sample will be by nucleic acid sequence analysis and may take the form of a sequencing technique. The Sanger dideoxynucleotide sequencing method is a well-known and widely used technique for sequencing nucleic acids. However more recently the so-called "next generation" or "second generation" sequencing approaches (in reference to the Sanger dideoxynucleotide method as the "first generation" approach) have become widespread. These newer techniques are characterised by high throughputs, e.g. as a consequence of the use of parallel, e.g. massively parallel sequencing reactions, or through less time-consuming steps. Various high throughput sequencing methods provide single molecule sequencing and employ techniques such as pyrosequencing, reversible terminator sequencing, cleavable probe sequencing by ligation, non-cleavable probe sequencing by ligation, DNA nanoballs, and real-time single molecule sequencing.

Nucleic acid sequence analysis may also preferably take the form of an oligonucleotide hybridisation probe based approach in which the presence of a target nucleotide sequence is confirmed by detecting a specific hybridisation event between a probe and its target. In these approaches the oligonucleotide probe is often provided as part of a wider array, e.g. an immobilised nucleic acid microarray. Preferably, the oligonucleotide probe sets and associated methods of WO2012080754 and WO2011043654 may be used to prepare microbiota profiles in accordance with the present invention.

In certain embodiments the methods of the invention may include steps in which the microbiota of a GI tract sample from the subject is profiled, e.g. by any of the above described techniques.

In certain embodiments the microorganisms or groups of microorganisms of which the relative levels thereof are to be determined are preselected, e.g. are microorganisms or groups of microorganisms which are indicator and/or causative species for the disease or condition of interest. This is however not essential so long as comparison within the method of the invention is made between the same microorganisms.

Thus, the term "microorganism" as used in the context of the invention may be any microbial organism, that is any organism that is microscopic, namely too small to be seen by the naked eye, which may be found in the GI tract. In particular, as used herein, the term includes the organisms typically thought of as microorganisms, particularly bacteria, fungi, archaea, algae and protists. The term thus particularly includes organisms that are typically unicellular, but which may have the capability of organising into simple cooperative colonies or structures such as filaments, hyphae or mycelia (but not true tissues) under certain conditions. The microorganism may be prokaryotic or eukaryotic, and may be from any class, genus or species of microorganism. Examples of prokaryotic microorganisms include, but are not limited to, bacteria, including the mycoplasmas, (e.g. Gram-positive, Gram-negative bacteria or Gram test non-responsive bacteria) and archaeobacteria. Eukaryotic microorganisms include fungi, algae and others that are, or have been, classified in the taxonomic kingdom Protista or regarded as protists, and include, but are not limited to, for example, protozoa, diatoms, protophyta, and fungus-like moulds.

In preferred embodiments the groups of microorganisms may be selected from Actinobacteria (e.g. Atopobium, Bifidobacterium), Bacteroidetes (e.g. Bacteroidia, e.g. Alistipes, Bacteroides, Prevotella, Parabacteroides, Bacteroidetes (in particular *Bacteroides fragilis*), Firmicutes (e.g. Bacilli, e.g. Bacillus, Lactobacillus, Pedicoccus, Streptococcus; Clostridia, e.g. Anaerotruncus, Blautia, Clostridium, Desulfitispora, Dorea, Eubacterium, Faecalibacterium, Ruminococcus; Erysipelotrichia, e.g. Catenibacterium, Coprobacillus, Unclassified Erysipelotrichaceae; Negativicutes, e.g. Dialister, Megasphaera, Phascolarctobacterium; Epsilonproteobacteria; Veillonella/Helicobacter (in particular *Dialister invisus, Faecalibacterium prausnitzii, Ruminococcus albus, Ruminococcus bromii, Ruminococcus gnavus, Streptococcus sanguinis, Streptococcus thermophilus*)), Proteobacteria (e.g. Gammaproteobacteria, e.g. Acinetobacter, Pseudomonas, Salmonella, Citrobacter, Cronobacter, Enterobacter, Shigella, Escherichia), Tenericutes (e.g. Mollicutes, e.g. Mycoplasma (in particular *Mycoplasma hominis*), and Verrucomicrobia (e.g. Verrucomicrobiae, e.g. Akkermansia (in particular *Akkermansia munciphila*)).

In the context of IBS, Firmicutes (Bacilli), Proteobacteria (*Shigella/Escherichia*), Actinobacteria and *Ruminococcus gnavus* may be important. Similarly, in the context of IBD, Proteobacteria (*Shigella/Escherichia*), Firmicutes, specifically *Faecalibacterium prausnitzii,* and Bacteroidetes (Bacteroides and Prevotella) may be important.

Thus, in certain embodiments references to microbiota profiles may be considered references to bacteriota profiles.

The number of microorgansims or groups of microorganisms of which the relative levels thereof are to be determined is not limited and so may be at least 2, 5, 10, 20, 30, 40, 50, 60, 70, 80, 90 or 100. In other embodiments the number may be less than 500, 200, 150, 100, 90, 80, 70, 60 or 50. Any range defined by endpoints of any of these numbers is hereby disclosed.

In certain embodiments the microbiota profiles may be normalised to account for inter-sample variation within each profiling experimental run and/or inter-experimental variation between each profiling experimental run through the use of appropriate controls during or after the analysis of the samples. Further normalisation to allow for batch variations in lab consumables and to correct for background signals may advantageously be performed.

In certain embodiments a centring vector may be applied to each microbiota profile element and/or each microbiota profile, wherein said vector is derived from the mean value for said microbiota profile element or microbiota profile obtained from corresponding samples from a plurality of normal subjects which have been profiled in the same way.

The test data matrix, if used, will typically be arranged such that each sample is presented as a single row and each microorganism or group of microorganisms of which the relative levels thereof have been determined is presented as a single column. The reciprocal of this arrangement may be used.

The term "latent variables" may refer to a subset of variables from within a data set which relate to potentially unknown correlations and trends. The term further includes variables which are determined from the combination of original variables in a data set (e.g. the level of a microorganism or group of microorganisms in a GI tract sample), specifically those that reflect correlations between variables and trends in the data set in a more meaningful way than the original variables. Thus, latent variables are typically derived from the algorithmic decomposition of a data set, e.g. by regression analysis, e.g. partial least squares regression analysis, principal components analysis (PCA), canonical correlation analysis, redundancy analysis, correspondence analysis, and canonical correspondence analysis. The latent variables may be orthogonal or non-orthogonal. Orthogonal latent variables are latent variables which are orientated perpendicular to one another. The latent variables of use in the invention are orthogonal latent variables.

Latent variables, in particular the orthogonal latent variables of use in accordance with the invention, may be determined by any convenient means, e.g. the partial least squares regression analysis of the levels of said plurality of microorganisms or groups of microorganisms in GI tract samples from a plurality of normal subjects, preferably which have been profiled in the same way. In certain embodiments the orthogonal latent variables are determined by the orthogonal transformation into principal components of the levels of said plurality of microorganisms or groups of microorganisms in GI tract samples from a plurality of normal subjects, preferably which have been profiled in the same way.

In preferred embodiments the orthogonal transformation into principal components is by is PCA, canonical correlation analysis, redundancy analysis, correspondence analysis, and canonical correspondence analysis.

In certain embodiments at least 2 vectors, e.g. at least 3, 5, 7, 9, 11, 13, 15, 17, 19 or 20 vectors are applied to the data set. In other embodiments no more than 50 vectors, e.g. no more than 40, 30, 25, 20, or 15 vectors are applied to the data set. Any range defined by endpoints of any of these numbers is hereby disclosed.

In accordance with the invention loading vectors are orthogonal latent variables determined by the regression analysis of the levels of a plurality of microorganisms or groups of microorganisms, e.g. by PCA, canonical correlation analysis, redundancy analysis, correspondence analysis, and canonical correspondence analysis, and so the number of loading vectors which may be used in accordance with the invention is limited by the number of microorganisms or groups of microorganisms investigated or the number of GI tract samples from the plurality of normal subjects of subjects with dysbiosis which have been profiled, whichever is the fewer: the greater the number of microorganisms or groups of microorganisms investigated and the greater the number of samples used the greater the number of orthogonal latent variables, and hence associated vectors, that may be present and may be selected from.

The determination of the orthogonal latent variables may be performed as part of the method of the invention, but more typically may be performed separately or the orthogonal latent variablesmay be obtained from other sources.

In certain embodiments the loading vector(s) are applied in the form of a projection matrix.

In preferred embodiments applying the loading vector(s) to the test data set comprises multiplying the profile elements of the data set by the loading vector(s), e.g. in the form of a projection matrix. Thus, this multiplication may be matrix multiplication.

In other embodiments, step (ii) in which a first projected data set is produced further comprises applying at least one eigenvalue determined from said at least one loading vector to the test data set. The eigenvalue may be applied before or after the loading vector or together with the loading vector. Applying said eigenvalue may comprise multiplying the eigenvalue with the profile elements of the test data set before or after application of the loading vector to the test data set. In other embodiments the eigenvalue may be multiplied with the loading vector before application to the test dataset, e.g. multiplication with the profile elements of the test data set. If appropriate in these embodiments, the references to an eigenvalue may include root, exponentiation or logarithm forms thereof. References to multiplication include matrix multiplication. In still further embodiments where a plurality of eigenvalues is applied in step (ii), the eigenvalues are applied in the form of a matrix with the eigenvalues arranged on the main diagonal, but this is not essential.

The normobiotic to dysbiotic threshold values to which the Q-residual of step (iv) and the Hotelling's $T^2$ of step (v) are compared are determined from the corresponding analysis of the same plurality of microorganisms or groups of microorganisms in a corresponding GI tract sample from a plurality of normal subjects and/or subjects with dysbiosis. The values displayed by these subjects will provide an indication of where the threshold between normobiosis and dysbiosis lies. The greater the number of subjects analysed the more accurately the threshold may be determined and preferably a plurality of both normal subjects and subjects with dysbiosis will be analysed in the determination of said normobiotic to dysbiotic threshold values. These thresholds represent the boundary between normobiosis and dysbiosis for a particular plurality of microorganisms or groups of microorganisms in a particular GI tract sample which have been profiled in a particular way and so will differ for each overall embodiment of invention and so must be prepared prior to the time at which the comparison with test samples is made.

Corresponding analysis means that the threshold values are determined using essentially the same means to process the profiling results from the corresponding GI tract sample as those used to prepare the Q-residual of step (iv) and the Hotelling's $T^2$ of step (v). Preferably corresponding analysis further means that the threshold values are determined using essentially the same profiling means on said corresponding samples.

More specifically said threshold values may be determined by:

(i) providing a normobiotic data set, wherein said normobiotic data set comprises at least one microbiota profile, said microbiota profile being a profile of the relative levels of a plurality of microorganisms or groups of microorganisms in a sample from the GI tract of a normal subject and wherein each level of each microorganism or group of microorganisms is a profile element of said data set,

(ii) applying to said first normobiotic data set the same at least one orthogonal latent variable determined by the regression analysis of the levels of said plurality of microorganisms or groups of microorganisms in corresponding GI tract samples from a plurality of normal subjects as applied to said test data set, thereby producing a first projected normobiotic data set,

(iii) providing said first projected normobiotic data set,

(iv) from said first projected normobiotic data set calculating the Q-residual of the microbiota profile ,

(v) from said first projected normobiotic data set calculating the Hotelling's $T^2$ for the microbiota profile from the variance explained by the orthogonal latent variables determined by the regression analysis of the levels of said plurality of microorganisms or groups of microorganisms in corresponding GI tract samples from a plurality of normal subjects of step (ii),

wherein step (v) may be performed before or after or concurrently with step (iv), and wherein said Q-residual and said Hotelling's $T^2$ are, or at least may contribute to, said respective normobiotic to dysbiotic thresholds.

More specifically said threshold values may also be determined by:

(i) providing a dysbiotic data set, wherein said dysbiotic data set comprises at least one microbiota profile, said microbiota profile being a profile of the relative levels of a plurality of microorganisms or groups of microorganisms in a sample from the GI tract of a subject with dysbiosis and wherein each level of each microorganism or group of microorganisms is a profile element of said data set,

(ii) applying to said first dysbiotic data set the same at least one orthogonal latent variable determined by the regression analysis of the levels of said plurality of microorganisms or groups of microorganisms in corresponding GI tract samples from a plurality of normal subjects as applied to said test data set, thereby producing a first projected dysbiotic data set,

(iii) providing said first projected dysbiotic data set,

(iv) from said first projected dysbiotic data set calculating the Q-residual of the microbiota,

(v) from said first projected dysbiotic data set calculating the Hotelling's $T^2$ for the microbiota profile from the variance explained by the orthogonal latent variables determined by the regression analysis of the levels of said plurality of microorganisms or groups of microorganisms in corresponding GI tract samples from a plurality of normal subjects of step (ii),

wherein step (v) may be performed before or after or concurrently with step (iv), and wherein said Q-residual and said Hotelling's $T^2$ are, or at least may contribute to, said respective normobiotic to dysbiotic thresholds.

Typically normobiotic to dysbiotic threshold values are selected to optimise class separation. Expressed differently, threshold values will be set such that values from at least 85%, e.g. at least 90%, 95%, 98% or 99% of the normal subjects analysed will lie on one side of the threshold and values from 85%, e.g. at least 90%, 95%, 98% or 99% of the subjects with dysbiosis that are analysed will lie on the other side of the threshold.

In embodiments in which the test data is quantitative or semi-quantitative the method of the invention may be performed in a way that provides a quantitative or semi-quantitative measure of the extent of dysbiosis or the extent of deviation from normobiosis. Such measures may be advantageous in the context of the diagnosis, prognosis and/or characterisation of diseases or conditions associated with dysbiosis since a greater extent of dysbiosis may indicate a more severe manifestation of the disease or condition or a particular subtype thereof. Such measures may also permit more accurate monitoring of disease progression by offering more comparative data.

In such embodiments the extent to which the Q-residual of step (iv) and the Hotelling's $T^2$ of step (v) differ from the normobiotic to dysbiotic threshold values to which they are compared will indicate (or provide a measure of, or be proportional to) the extent of dysbiosis.

In more specific embodiments dysbiosis may be quantified by combining the Q-residual of step (iv) and the Hotelling's $T^2$ of step (v) into a single metric for dysbiosis. This second combination may be achieved by any convenient means that results in a meaningful informational output from this step. The second combination may simply be the sum or multiplication of the Q-residual of step (iv) and the Hotelling's $T^2$ of step (v).

Thus the invention provides a method for quantifying dysbiosis, said method comprising performing the above described method of the invention, wherein said comparisons with normobiotic to dysbiotic thresholds together comprise combining the Q-residual of step (iv) and the Hotelling's $T^2$ of step (v) into a single metric for dysbiosis.

In other embodiments further manipulation may occur, e.g. the application of root, exponentiation or logarithm techniques to the Q-residual of step (iv) and/or the Hotelling's $T^2$ of step (v). In further embodiments weightings may be applied to one or both of these combinations (discussed in more detail below). In still further embodiments the second combination may also be manipulated further. In still further embodiments the Euclidean distance from the origin for both the Q-residual of step (iv) and the Hotelling's $T^2$ of step (v) is calculated. Thus, in certain embodiments the Q-residual of step (iv) and the Hotelling's $T^2$ of step (v) are both squared, then summed and then the square root of that calculation is determined to give said single metric for dysbiosis. This may be represented as the following formula (Formula I) wherein Qres represents the Q-residual of step (iv) and $T^2$ represents the Hotelling's $T^2$ of step (v).

Formula I:

$$r = \sqrt{\left\{T^2\right\}^2 + Qres^2}$$

A single metric is highly convenient and offers advantages in the interpretation of results from different subjects. Surprisingly, it has been found that the Q-residual of step (iv) and the Hotelling's $T^2$ of step (v) are potentially correlated and that a high value for one combination can be associated with a high value of the other. It has been recognised that simply summing these values may over-represent the extent of dysbiosis and so the use of Euclidean distance may be advantageous as it reduces the risk of the single metric over-representing the extent of dysbiosis posed by the simple addition of values in the second combination.

A normobiotic to dysbiotic threshold for the same plurality of microorganisms or groups of microorganisms in a corresponding GI tract sample expressed in the same terms as the above described metric may be calculated in the same way from a plurality of normal subjects and/or subjects with dysbiosis and the extent to which the metric for a test sample differs from said threshold will be proportional to the extent of dysbiosis.

In preferred embodiments the combining of the Q-residual of step (iv) and the Hotelling's $T^2$ of step (v) into a single metric for dysbiosis will comprise scaling said Q-residual of step (iv) and the Hotelling's $T^2$ of step (v) to result in values of similar magnitude, e.g. by dividing the Q-residual of step (iv) and the Hotelling's $T^2$ of step (v) by their respective normobiotic to dysbiotic class thresholds (i.e. the normobiotic to dysbiotic thresholds to which comparison is made in steps (iv) and (v) respectively). This may be represented as the following formula (Formula II) wherein q represents the Q-residual of step (iv) and $T^2$ represents the Hotelling's $T^2$ of step (v).

Formula II:

$$r = \sqrt{\left(\frac{q}{q_{thres}}\right)^2 + \left(\frac{T^2}{T^2_{thres}}\right)^2}$$

A scaled single metric is highly convenient and offers advantages in the interpretation of results from different subjects.

In still further embodiments, in order to quantify dysbiosis, said single metric (preferably said scaled single metric) may further be plotted on a finite, preferably continuous, numerical scale from normobiotic to dysbiotic (or vice versa) with class separation (the boundary between normobiosis and dysbiosis) at a predetermined point, preferably a predetermined integer value, on that finite numerical scale which represents, or is, a combination of, preferably the sum of, the normobiotic to dysbiotic class thresholds (similarly scaled if appropriate) of steps (iv) and (v).

The extent to which a said single metric determined for a test sample differs from the class separation point in the direction of the maximum dysbiotic endpoint of the scale is proportional to the extent of dysbiosis. Preferably, the portion of the numerical scale between the class separation point and the maximum dysbiotic endpoint of the scale is subdivided into discrete regions which further quantify dysbiosis. Preferably said regions have boundaries at defined points, e.g. numerical integers.

Thus the invention provides a method for quantifying dysbiosis, said method comprising performing the above described method of the invention and further plotting said single metric for dysbiosis on a finite, preferably continuous, numerical scale with class separation at a predetermined point which represents, or is, a combination of the normobiotic to dysbiotic class thresholds of steps (iv) and (v), similarly scaled if scaling has been applied.

Similarly, the extent to which a said single metric determined for a test sample differs from the class separation point in the direction of the maximum normobiotic endpoint of the scale is inversely proportional to the extent to which the test sample deviates from the model definition of normal. To help visualise this, the portion of the numerical scale between the class separation point and the maximum normobiotic endpoint of the scale may be subdivided into discrete regions which further quantify deviation from the model definition of normal. Preferably said regions have thresholds at defined points, e.g. numerical integers.

For ease of interpretation, the single metric may be reported in terms of the nearest threshold between the various regions of the scale or class separation point.

Plotting said single metric on such a numerical scale, i.e. a scale with a class separation point that represents, or is, a combination of, preferably the sum of, the normobiotic to dysbiotic class threshold values of steps (iv) and (v), ensures both elements of the metric contribute to the finding of the extent of dysbiosis.

As a result of the fact that said single metric is a combination of two different measures (the Q-residual of step (iv) and the Hotelling's $T^2$ of step (v) the class boundary (normobiosis to dysbiosis) defined by these measures is two dimensional and simply summing the associated normobiotic to dysbiotic class threshold values for both measures to determine the class separation point on said finite numerical scale may not be able to fully resolve the variation between results from normobiotic and dysbiotic samples which may be seen at or close to the class boundary when only one of these measures is beyond its respective threshold value. It may therefore be more accurate, when further plotting the single metric on a finite numerical scale from normobiotic to dysbiotic (or vice versa) with class separation (the boundary between normobiosis and dysbiosis) at a predetermined point, to set the predetermined class separation point differentially depending on whether or not said test sample has at least one of these measures beyond their respective threshold value. In these embodiments, the class separation point for a test sample having at least one of the Q-residual of step (iv) or the Hotelling's $T^2$ of step (v) above the normobiotic to dysbiotic class threshold values of steps (iv) and (v), respectively, will correspond to, or preferably be, that of one or other of the exceeded normobiotic to dysbiotic class threshold values of steps (iv) or (v). Also in these embodiments the class separation point for a test sample in which neither of the Q-residual of step (iv) or the Hotelling's $T^2$ of step (v) are beyond the normobiotic to dysbiotic threshold values of steps (iv) and (v), respectively, will correspond to, or preferably be, the sum of the normobiotic to dysbiotic class thresholds of steps (iv) and (v). In these embodiments "corresponds to" includes root, exponentiation or logarithm forms thereof, preferably the square root form of said threshold values.

Thus the invention provides a method for quantifying dysbiosis, said method comprising performing the above described method of the invention and further plotting the single metric on a finite numerical scale with class separation at a predetermined point, wherein for a test sample having at least one of the Q-residual of step (iv) or the Hotelling's $T^2$ of step (v) above the normobiotic to dysbiotic class threshold values of steps (iv) and (v), respectively, said class separation point will correspond to that of one or other of the exceeded normobiotic to dysbiotic class threshold values of steps (iv) or (v) and for a test sample in which neither of the Q-residual of step (iv) or the Hotelling's $T^2$ of step (v) are beyond the normobiotic to dysbiotic threshold values of steps (iv) and (v), respectively, said class separation point will correspond to the sum of the normobiotic to dysbiotic class thresholds of steps (iv) and (v).

In these embodiments it may be convenient if a representative value is applied to the class separation point which remains consistent regardless of the true value of the class separation point applicable to the test sample in question, thus facilitating the use of the same scale to report results from both normobiotic or dysbiotic subjects.

In embodiments in which a portion of the numerical scale between the class separation point and the maximum dysbiotic endpoint of the scale is subdivided into discrete regions which further quantify dysbiosis in terms of defined numerical integers, it may be advantageous to calculate where said single metric falls more precisely as a decimal value between said integer values, thereby a more accurate quantification of dysbiosis may be achieved. In such embodiments calculation of said decimal values between said integers is done via a density or probability distribution function. Numerous techniques are available to the skilled person who would be able to select from or combine such techniques to meet his/her needs or to design new techniques. By way of non-limiting examples the following distributions may be used: lognormal distribution, continuous uniform distribution, discrete uniform distribution, normal (or Gaussian) distribution, student's t-distribution, chi-squared distribution, F-distribution, logit normal distribution, log-logistic distribution, Pareto distribution, Bernoulli distribution, binomial distribution, geometric distribution, Poisson distribution, exponential distribution, gamma distribution, beta distribution. In these embodiments the single metric may still be reported in terms of the nearest threshold between the various regions of the scale or class separation point but, by calculating the decimal value, determining which threshold is nearest will be more accurate.

In preferred embodiments, the effects of technical variation on the plotting of test data onto said finite numerical scale, in particular between said class separation point and said maximum dysbiosis endpoint, is minimised by applying weightings to the Q-residual of step (iv) and/or the Hotelling's $T^2$ of step (v) during the second combination step. Where weightings are applied to both combinations, the weightings may be the same or may differ. One or other may be zero. Suitable weightings may be determined without undue burden by repeatedly analysing a reference sample or a plurality of reference samples, e.g. a sample or samples from a subject with GI tract dysbiosis, in accordance with the invention and the determining what weightings values, if any, for each combination result in the most stable inter-experimental output.

In preferred embodiments, this may be expressed as the following formula (Formula III) wherein q represents the Q-residual of step (iv), $T^2$ represents the Hotelling's $T^2$ of step (v) and w represents the weighting applied.

Formula III

$$r = \sqrt{w_q * \left(\frac{q}{q_{thres}}\right)^2 + w_{T^2} * \left(\frac{T^2}{T^2_{thres}}\right)^2}$$

In certain embodiments weightings may be applied to the analysis of samples having at least one of the Q-residual of step (iv) or the Hotelling's $T^2$ of step (v) in excess of the normobiotic to dysbiotic class threshold values of steps (iv) and (v), respectively, but not to the analysis of a test sample in which neither of the Q-residual of step (iv) or the Hotelling's $T^2$ of step (v) are beyond the normobiotic to dysbiotic threshold values of steps (iv) and (v), respectively.

In certain embodiments the methods of the invention do not comprise a step in which a microbiota profile from the GI tract sample is compared, e.g. directly, to a corresponding profile representative of a particular disease or condition or stage thereof or to a corresponding profile representative of a healthy subject or a patient with GI tract dysbiosis..

As discussed above, many diseases and conditions, or stages thereof, are believed to be associated with perturbations in the microbiota of the GI tract, or regions thereof. The above described methods of the invention, in particular the quantitative or semi-quantitative methods, may therefore be used to obtain and provide information relevant to the diagnosis, monitoring and/or characterisation of diseases and conditions associated with perturbations in the microbiota of the GI tract or the assessment of the risk of developing a disease or condition which is associated with a perturbation of the microbiota profile of the GI tract. As is clear from the discussion herein, perturbation of the microbiota profile of the GI tract may be considered GI tract dysbiosis.

Thus in a further aspect the invention provides a method for obtaining information relevant to the diagnosis, monitoring and/or characterisation of diseases and conditions associated with perturbations in the microbiota of the GI tract or the assessment of the risk of developing a disease or condition which is associated with by a perturbation of the microbiota profile of the GI tract, said method comprising performing a method as defined above, wherein the product of said method as defined above, the indication of the likelihood of dysbiosis or the extent of dysbiosis, provides said information.

In a further aspect the invention provides a method for diagnosing, monitoring and/or characterising diseases and conditions associated with perturbations in the microbiota of the GI tract or the assessing of the risk of developing a disease or condition which is associated with a perturbation of the microbiota profile of the GI tract, said method comprising performing a method as defined above wherein the indication of the likelihood of dysbiosis or the extent of dysbiosis is indicative of the presence or absence, the risk of developing, the progress of, or the characteristics of said disease or condition associated with perturbations in the microbiota of the GI tract. In these embodiments the method may further comprise a step of making a diagnosis, of monitoring and/or of making a characterisation of a disease or condition associated with perturbations in the microbiota of the GI tract or of making an assessment of the risk of developing a disease or condition which is associated with a perturbation of the microbiota profile of the GI tract based on the indication of the likelihood or the extent of dysbiosis provided in the preceding steps. The results of such a latter step may be recorded and optionally stored on a suitable recording/storage medium and/or communicated to a physician, the subject or intermediary or agent thereof.

In certain embodiments said method is performed with microbiota profiles from a plurality of GI tract samples taken from the patient at different time points. In this way changes in the subjects GI tract microbiota over time may be investigated.

Diseases and conditions associated with perturbations in the microbiota of the GI tract, i.e. dysbiosis, may be considered to be those which may be caused by, or exacerbated by, a shift in the profile of the microbiota of the GI tract (or regions thereof) those which cause, or result in, the display of a profile of the microbiota of the GI tract tract (or regions thereof) that differs from the normal state or those which may be characterised by or identified by the display of a profile of the microbiota of the GI tract tract (or regions thereof) that differs from the normal state. Examples of such diseases and conditions include, but are not limited to, functional GI tract disorders, e.g. Inflammatory Bowel Disease (IBD), Crohn's Disease (CD), Ulcerative Colitis (UC), Irritable Bowel Syndrome (IBS) and dyspepsia; small bowel bacterial overgrowth syndrome and GI tract cancers (e.g. cancer of the mouth, pharynx, oesophagus, stomach, duodenum, jejunum, ileum, cecum, colon, rectum or anus); breast cancer; ankylosing spondylitis; non-alcoholic steatohepatitis; atopic diseases, e.g. eczema, asthma, atopic dermatitis, allergic conjunctivitis, allergic rhinitis and food allergies; metabolic disorders, e.g. diabetes mellitus (type 1 and type 2), obesity and metabolic syndrome; neurological disorders, e.g. depression, multiple sclerosis, dementia, and Alzheimer's disease; autoimmune disease (e.g. arthritis); malnutrition; chronic fatigue syndrome and autism. In preferred embodiments the methods of the invention are performed in the context of IBS.

"Diagnosis" refers to determination of the presence or existence of a disease or condition or stage thereof in an organism. "Monitoring" refers to establishing the extent of, or possible changes in, a disease or condition, particularly when an individual is known to be suffering from a disease or condition, for example to monitor the effects of treatment or the development of a disease or condition, e.g. to determine the suitability of a treatment, to provide a prognosis, and/or to determine if a patient is in remission or relapse. "Characterising" includes determining the features of a particular disease or condition of a subject, e.g. the extent or severity of the disease or condition or the subtype thereof, including likelihood to respond to particular therapies.

"Assessing the risk of a subject developing a disease or condition" refers to the determination of the chance or the likelihood that the subject will develop the disease or condition. This may be expressed as a numerical probability in some embodiments. The assessment of risk may be by virtue of the extent of dysbiosis determined by the methods of the invention.

"Disease" refers to a state of pathological disturbance relative to normal which may result, for example, from infection or an acquired or congenital genetic imperfection.

A "condition" refers to a state of the mind or body of an organism which has not occurred through a recognised disease, e.g. the presence of an agent in the body such as a toxin, drug or pollutant, or pregnancy.

"Stage thereof" refers to different stages of a disease or condition which may or may not exhibit particular physiological or metabolic changes, but do exhibit changes in the profile of the GI tract microbiota. In some embodiments the observed differences in the profile of GI tract microbiota may lead to a previously unappreciated classification of the progress of a disease or condition.

The subject may be any human or non-human animal subject, but more particularly may be a vertebrate, e.g. a mammal, including livestock and companion animals. Preferably the subject is a human, in which case the term "patient" may be used interchangeably with the term "subject". The subject may be of any age, e.g. an infant, a child, a juvenile, an adolescent or an adult, preferably an adult. In humans, an adult is considered to be of at least 16 years of age and an infant to be up to 2 years of age. In certain embodiments the subject will be an infant, in others it will be a child or an adult. The subject may have or be suspected of having or be or suspected of being at risk of dysbiosis, or the medical condition in question (e.g. IBS and IBD and its subcategories CD and UC).

A "normal" or "healthy" subject is a subject that is not considered to have the illness or disease or other medical condition, the diagnosis of which is the object of the method in question, or a disease, illness or other medical condition that is similar thereto or shares common features and symptoms, e.g. GI symptoms and features. A "normal" or "healthy" GI tract is a GI tract from such subjects. Alternatively put, a "normal" or "healthy" subject (or GI tract) is a subject/GI tract that does not have GI tract dysbiosis. In other embodiments a normal or healthy subject will be essentially free of serious illness or disease or other medical conditions, or at least is a subject that does not have observable or detectable symptoms of any recognised serious illness or disease. In other embodiments a normal or healthy subject will be free of all illness or disease or other medical conditions, or at least does not have observable or detectable symptoms of any recognised illness or disease. Preferably these references to illness, disease or medical condition is a reference to an illness, disease or medical condition of the GI tract.

The word "corresponding" is used to convey the concept that the subject to which the term is applied is the same as another instance of that subject. Thus the essential features that define that subject are shared by the other subject even though precise details may be unique. Alternative terms could be "matching", "analogous", agreeing", "equivalent" or "same as".

The methods of the invention may be performed on a computer, system or apparatus carrying a program adapted to perform said methods or at least one of the steps thereof. Thus, the methods of the invention are computer-implemented methods and the invention further provides a computer, system or apparatus carrying a program adapted to perform the methods of the invention. Typically a processor for executing the software and a storage device for storing the test data and the results of one or more steps of the methods of the invention will be present. The processor and storage device will typically be in communication with one another. In one embodiment, the computer, system or apparatus is in communication with a network, such as the Internet, e.g. for communication with laboratories and clinics that communicate test data and/or receive the output of the methods of the invention. The system or apparatus may be further adapted to perform microbiota profiling, or a step thereof, e.g. the step that results in a microbiota profile of use in accordance with the present invention, preferably in a partial or fully automated manner. The invention further provides a computer readable medium carrying said program and such a program per se. In still further aspects the invention provides Formulae I, II and III and the use thereof in the types of methods described generally herein and the specific methods of the invention recited herein.

The results (final output) from the methods of the invention may be provided on computer or human readable media or communicated by any suitable means, electronic or otherwise, for comprehension and/or further interpretation by a skilled person.

The methods of the invention may be used alone as an alternative to other investigative techniques or in addition to such techniques in order to provide information on the microbiota profiles of a subject, e.g. in the diagnosis etc. of diseases and conditions associated with perturbations in the microbiota of the GI tract, in particular in order to diagnose IBS or to dismiss IBS as an explanation for the symptoms presented by the subject. In the context of the diagnosis of IBS, for example, the methods of the invention may be used as an alternative or additional diagnostic measure to diagnosis using imaging techniques such as Magnetic Resonance Imaging (MRI), ultrasound imaging, nuclear imaging, X-ray imaging or endoscopy or IBD serological markers, e.g. anti-Saccharomyces *cerevisiae* antibodies (ASCA) and peri-nuclear anti-neutrophil cytoplasmic antibodies (pANCA).

In the methods described above may be combined with the therapeutic treatment of said subject in a manner consistent with the diagnosis or prognosis made to alleviate, reduce, remedy or modify at least one symptom or characteristic of the disease or condition associated with perturbations in the microbiota of the GI tract that the subject has (e.g. IBS etc.), e.g. by administering a pharmaceutical composition (which may be considered to include faecal microbotal transplants and microbial cultures) and/or performing a surgical procedure appropriate to treat the disease or condition and/or adjusting the lifestyle of the subject in a manner appropriate to treat the disease or condition. In this regard, the invention can be considered applied in the context of methods for the therapeutic treatment of diseases or conditions associated with perturbations in the microbiota of the GI tract (e.g. IBS etc.) and for guiding and/or optimising such treatments. This may include treatments to remedy, or at least address in part, the GI tract dysbiosis of a subject or the extent thereof.

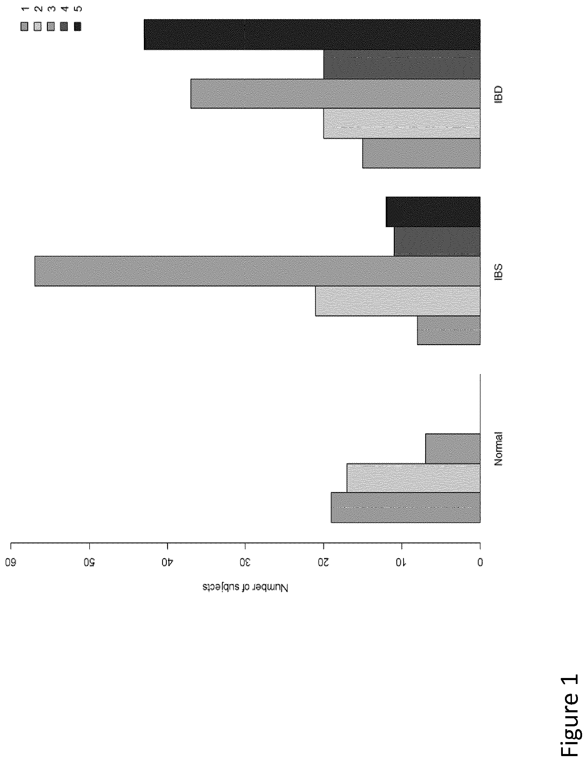

**Figure 1** shows distribution of DI scores 1-5 for the validation cohort as determined in Example 1, showing the increase in DI from normal individuals through IBS patients and finally in IBD patients.

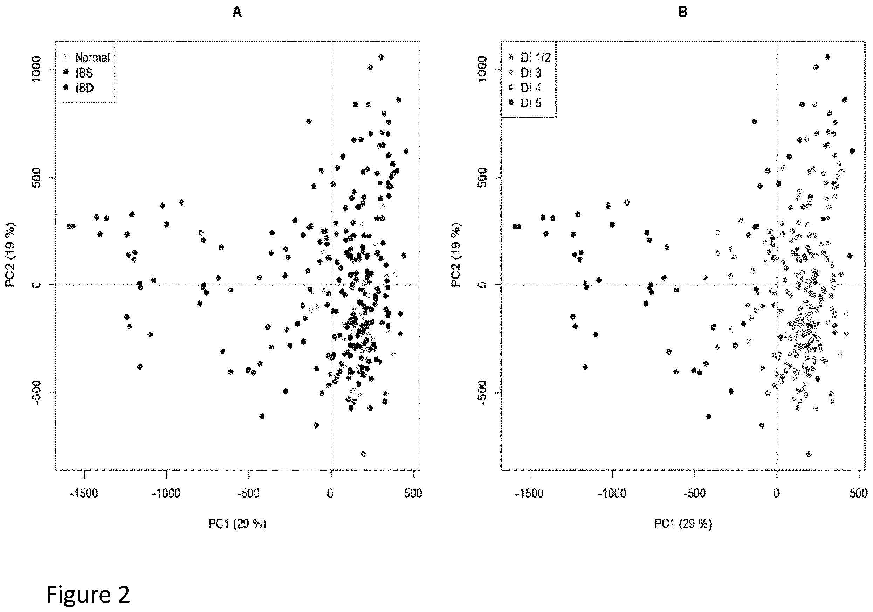

**Figure 2** shows PCA scores for the first two principal components for validation cohort (n = 287) based on 54 probes. The two PCs account for 48% of the variation, and points are coloured according to **A)** cohort: yellow - normal, blue - IBS, and red - IBD; and **B)** DI: grey = 1-2, orange = 3, red = 4, dark red = 5.

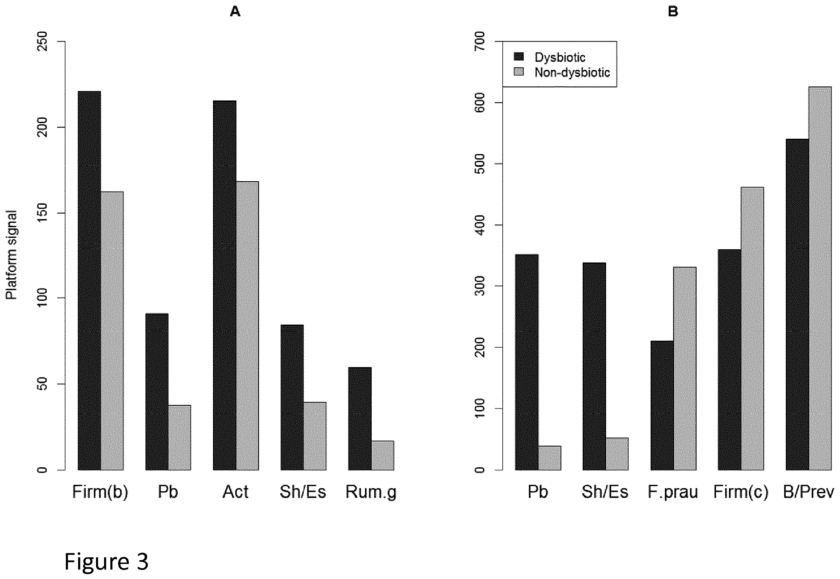

**Figure 3** shows mean normalised signal for top five probes sorted by absolute relative difference between dysbiotic (red) and non-dysbiotic (grey) as determined in Example 1 for **A)** IBS patients (n = 109), and **B)** IBD patients (n = 135). Act; Actinobacteria, B/Prev; Bacteroides/Prevotella, Firm(b); Firmicutes (Bacilli), Firm (c); Firmicutes (Clostridia), F. prau; *Faecalibacterium prausnitzii,* Pb; Proteobacteria, Rum.g; Ruminococcus gnavus, Sh/Es; *Shigella/Escherichia.*

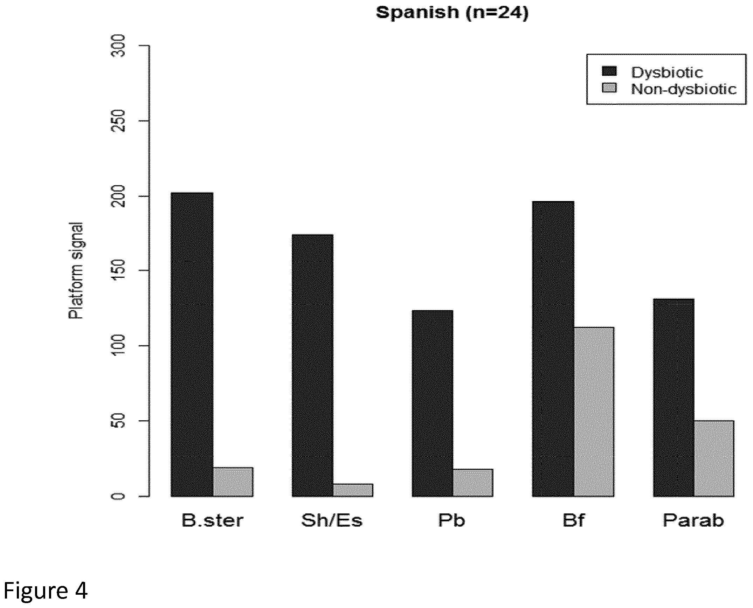

**Figure 4** shows mean normalised signal for probes sorted by absolute relative difference between dysbiotic (red) and non-dysbiotic (grey) as determined in Example 1 for Spanish cohort (n = 24). Bf; Bifidobacterium, B.ster; Bacteroides stercoris, Parab; Parabacteroides, Pb; Proteobacteria, Sh/Es; *Shigella/Escherichia.*

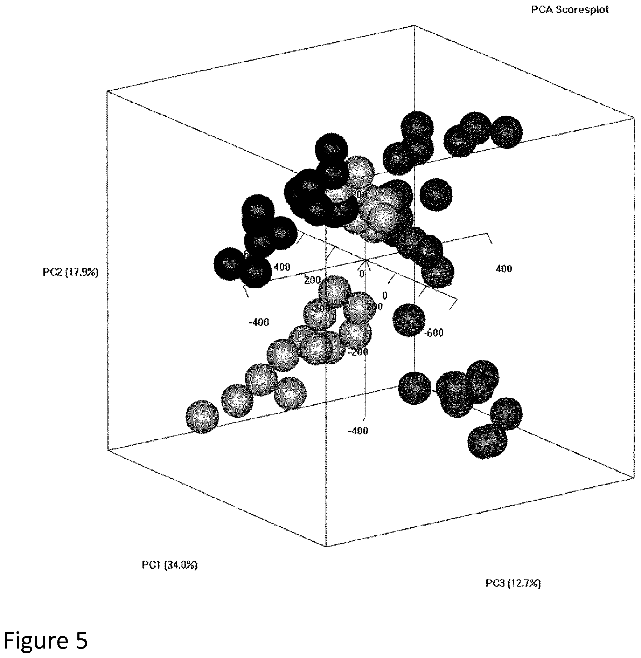

**Figure 5** shows scores for the first three principal components from PCA of normalised data from five healthy subjects collected weekly for up to 14 weeks (n = 64). One point is one sample for donor x taken at time point y. The first three PCs account for 65% of the variation, and points are coloured according to donor.

**EXAMPLE 1** - **Preparing profiles of GI tract microbiota with 54 probes targeting a plurality of microorganisms or groups of microorganisms and using same to determine dysbiosis in IBS and IBD patients**

**Materials and methods**

Human samples

Faecal samples were collected from 668 adults (aged 17-76; 69% women), including normal individuals (*n* = 297) and patients with IBS (*n* = 236) and IBD (*n* = 135) (Table 1). Faecal samples were collected from hospitals in Norway, Sweden, Denmark and Spain (72%), as well as from workplaces in Oslo, Norway (28%), in an effort to achieve heterogeneity. The normal donors had no clinical signs, symptoms or history of IBD, IBS or other organic gastrointestinal-related disorders (e.g. colon cancer). The IBS samples were collected as part of prospective studies that used the Rome III diagnostic criteria to identify IBS. The distribution of IBS subtypes was 44% IBS-diarrhoea, 22% IBS-alternating, 17% IBS-constipation, 11% IBS-un-subtyped, and 4% IBS-mixed. The diagnosis of IBD was based on clinical presentation confirmed by colonoscopy. Of the 135 IBD samples, 80 (59%) were treatment-naive patients and 55 (41%) were IBD patients in remission. The distribution of IBD types was 62% UC and 38% CD for the treatment-naive group, and 67% UC and 33% CD for the IBD in remission group. Informed consent was obtained for all samples along with approval from local scientific ethics committees.

**Table 1. Demographic information**

| Categories | Total | Females, % | Age (years)* | |
| | | | Mean | Range |
|---|---|---|---|---|
| Normal controls | 297 | 63 | 41 | 21-70 |
| Nordic | 254 | 64 | 42 | 21-70 |
| Danish | 19 | 63 | 42 | 23-61 |
| Spanish | 24 | 50 | 35 | 22-56 |
| IBS† | 236 | 78 | 40 | 17-76 |
| IBS-D | 102 | 79 | 40 | 18-70 |
| IBS-C | 41 | 85 | 42 | 22-73 |
| IBS-M | 10 | 80 | 37 | 19-55 |
| IBS-U | 25 | 88 | 41 | 19-68 |
| IBS-A | 51 | 67 | 39 | 20-62 |
| IBD treatment-naive | 80 | 56 | 34 | 18-61 |
| CD | 30 | 50 | 33 | 19-53 |

(continued)

| Categories | Total | Females, % | Age (years)* | |
| --- | --- | --- | --- | --- |
| | | | Mean | Range |
| UC | 50 | 63 | 35 | 18-61 |
| IBD remission‡ | 55 | 76 | 42 | 20-69 |
| CD | 18 | 72 | 38 | 20-59 |
| UC | 36 | 78 | 44 | 24-69 |

A, alternating; C, constipation; CD, Crohn's disease; D, diarrhoea; IBS, irritable bowel syndrome; IBD, inflammatory bowel disease; M, mixed; U, un-subtyped; UC, ulcerative colitis.
*Precise ages were known for 99% of the total samples used.
†IBS type known for 97% of the total IBS samples used.
‡CD/UC diagnosis known for 99% of the total IBD samples used.

Sample collection

Samples were collected at home, office or hospital, and frozen within 3-5 days. Faecal samples were mixed with stool transport and recovery buffer (Roche, Basel, Switzerland) in a 1:3 ratio by vortexing. All samples were pulse centrifuged and 600 $\mu$l was transferred to a 96-well Lysing Matrix E rack (MP Biomedicals Inc., Santa Ana, CA, USA). Samples were mechanically lysed twice at 1800 rpm, 40 s on 40 s rest, in a FastPrep-96™ (MP Biomedicals Inc.). Lysed samples were centrifuged (5 min, 1300 g, PlateSpin II centrifuge, Kubota, Tokyo, Japan), and 250 $\mu$l was incubated at 65°C for 15 min with 250 $\mu$l lysis buffer BLM and 20 $\mu$l protease. A 400$\mu$l aliquot of each protease-treated faecal sample was used to extract total genomic DNA according to mag™ maxi kit instructions (LGC Genomics, Berlin, Germany), adjusted for a MagMAX™ express 96 DNA extraction robot (Life Technologies, Waltham, MA, USA).
The polymerase chain reaction (PCR) primers (targeted the 16S rRNA gene and were used to amplify 1180 base pair fragments containing seven variable regions (V3-V9). This was followed by a reaction clean-up as described by Vebø et al (Vebo HC, Sekelja M, Nestestog R, et al. Temporal development of the infant gut microbiota in immunoglobulin E-sensitized and nonsensitized children determined by the GA-map infant array. Clin Vaccine Immunol 2011; 18: 1326-1335) with minor modifications.

Sample analysis: SNE, hybridisation and detection

The PCR template (>75 ng) was used in an single-nucleotide extension (SNE) reaction described in Vebø et al, with the following modifications: a final volume of 25 $\mu$l containing 0.5 $\mu$M BIOTIN-11-ddCTP (Perkin Elmer, Waltham, MA, USA) was used through five labelling cycles to label a probe-set of 55 probes (0.01 $\mu$M) (54 bacteria target probes and Universal control). Complementary probes coupled to carboxyl barcoded magnetic beads (BMBs, Applied BioCode, Santa Fe Springs, CA, USA) were hybridised to the SNE probes and quantified using a BioCode 1000A Analyzer (Applied BioCode). In brief, a 10 $\mu$l SNE sample was added to a 40 $\mu$l reaction volume containing 31.2 $\mu$l BMB buffer, hybridisation control and 1.8 $\mu$l coupled BMBs. Samples were incubated at 700 rpm, 95°C for 3 min, followed by 700 rpm, 45°C for 15 min in a Vortemp™ 56 (Labnet International Inc., Edison, NJ, USA). A 25 $\mu$l BMB buffer containing 20 $\mu$g/ml streptavidin R-phycoerythrin LumiGrade ultrasensitive reagent (Roche) was added to each sample before 90 minutes of incubation at 700 rpm, 45°C. Finally, samples were washed according to Applied BioCode's recommendations. The hybridisation signal was processed by the BioCode 1000A Analyzer software (Applied BioCode). The software identified and quantified median signals, bead count and flags, and raw data files were exported for further analysis.

Probe identification, selection, *in silico* and *in vitro* testing

To establish and optimise the most applicable bacterial probe set, data from previous IBD and IBS intestinal microbiota research was compiled based on predefined search criteria to provide >500 bacterial observations associated with the occurrence of IBD and IBS. From a combined dataset of 496 16S rRNA gene sequences (consensus sequence[s] for each species, chosen from all available long 16S rRNA sequences and purified to avoid sequences errors) from 269 bacterial species, probes were designed to cover major bacterial observations made from the literature. All probes were designed according to Vebø et al with a minimum melting temperature ($T_m$) of 60°C by the nearest-neighbour method for the target group where the nucleotide 3' end of the probe is a cytosine; non-target group probe requirements were a $T_m$ of 30°C or absence of a cytosine as the nucleotide adjacent to the 3' end of the probe. Each probe was designed to target a bacterial species or group, i.e. *Faecalibacterium prausnitzii* (species), Lactobacillus (genus), Clostridia (class) and Proteobacteria (phylum), based on their 16S rRNA sequence (V3-V9). Probes that satisfied target detection and non-target exclusion *in silico* were evaluated for cross-labelling, self-labelling and cross hybridisation before final validation was performed against bacterial strains *in vitro.*

After *in vitro* testing, a panel of 124 optimal probes was further selected using variable selection methods: variable importance in projection, selectivity ratio and interval partial least squares using data from a selection of normal and IBS samples (data not shown). The variables (probes) were selected based on their ability to distinguish between samples isolated from normal healthy individuals and IBS patients. A final panel of 54 probes was selected covering the sites across V3 to V7 on the 16S rRNA sequence. Bacterial target specificity, tested with the 54-probe set against 368 single bacterial strains was performed to define the target bacteria for each probe. As shown in Table 2, the probes detect bacteria within the six phyla; Firmicutes, Proteobacteria, Bacteroidetes, Actinobacteria, Tenericutes and Verrucomicrobia, covering 11 taxonomic bacterial classes and 36 genera.

**Table 2: List of the bacterial targets of the 54 labelling probes**

| Probe number | Phylum | Class | Genus |
|---|---|---|---|
| 1 | Actinobacteria | Actinobacteria | *Atopobium* |
| 2 | Actinobacteria | Actinobacteria | *Bifidobacterium* |
| 3 | Actinobacteria | Actinobacteria | |
| 4 | Actinobacteria | | |
| 5 | Bacteroidetes | Bacteroidia | *Alistipes* |
| 6 | Bacteroidetes | Bacteroidia | *Alistipes* |
| 7 | Bacteroidetes | Bacteroidia | *Bacteroides* |
| 8 | Bacteroidetes | Bacteroidia | *Bacteroides/Prevotella* |
| 9 | Bacteroidetes | Bacteroidia | *Bacteroides* |
| 10 | Bacteroidetes | Bacteroidia | *Bacteroides* |
| 11 | Bacteroidetes | Bacteroidia | *Bacteroides* |
| 12 | Bacteroidetes | Bacteroidia | *Bacteroides* |
| 13 | Bacteroidetes | Bacteroidia | *Parabacteroides* |
| 14 | Bacteroidetes | Bacteroidia | *Parabacteroides* |
| 15 | Bacteroidetes | Bacteroidia | *Prevotella* |
| 16 | Firmicutes | Bacilli | *Bacillus* |
| 17 | Firmicutes | Bacilli | *Lactobacillus* |
| 18 | Firmicutes | Bacilli | *Lactobacillus* |
| 19 | Firmicutes | Bacilli | *Pedicoccus/Lactobacillus* |
| 20 | Firmicutes | Bacilli | *Streptococcus* |
| 21 | Firmicutes | Bacilli | *Streptococcus* |
| 22 | Firmicutes | Bacilli | *Streptococcus* |
| 23 | Firmicutes | Bacilli | *Streptococcus* |
| 24 | Firmicutes | Bacilli | |
| 25 | Firmicutes | Bacilli/Clostridia | *Streptococcus/Eubacterium* |
| 26 | Firmicutes | Clostridia | *Anaerotruncus* |
| 27 | Firmicutes | Clostridia | *Blautia* |
| 28 | Firmicutes | Clostridia | *Clostridium* |
| 29 | Firmicutes | Clostridia | *Clostridium* |
| 30 | Firmicutes | Clostridia | *Desulfitispora* |
| 31 | Firmicutes | Clostridia | *Dorea* |
| 32 | Firmicutes | Clostridia | *Eubacterium* |
| 33 | Firmicutes | Clostridia | *Eubacterium* |
| 34 | Firmicutes | Clostridia | *Eubacterium* |
| 35 | Firmicutes | Clostridia | *Faecalibacterium* |
| 36 | Firmicutes | Clostridia | *Ruminococcus* |
| 37 | Firmicutes | Clostridia | |
| 38 | Firmicutes | Clostridia | |

(continued)

| Probe number | Phylum | Class | Genus |
|---|---|---|---|
| 39 | Firmicutes | Erysipelotrichia | *Catenibacterium* |
| 40 | Firmicutes | Erysipelotrichia | *Coprobacillus* |
| 41 | Firmicutes | Erysipelotrichia | Unclassified Erysipelotrichaceae |
| 42 | Firmicutes | Negativicutes | *Dialister* |
| 43 | Firmicutes | Negativicutes | *Megasphaera/Dialister* |
| 44 | Firmicutes | Negativicutes | *Phascolarctobacterium* |
| 45 | Firmicutes | Negativicutes | |
| 46 | Firmicutes | Negativicutes/ Epsilonproteobacteria/ Clostridia | *Veillonella/Helicobacter* |
| 47 | Firmicutes/ Tenericutes/ Bacteroidetes species | | |
| 48 | Proteobacteria | Gammaproteobacteria | *Acinetobacter* |
| 49 | Proteobacteria | Gammaproteobacteria | *Pseudomonas* |
| 50 | Proteobacteria | Gammaproteobacteria | *Salmonella, Citrobacter, Cronobacter, Enterobacter* |
| 51 | Proteobacteria | Gammaproteobacteria | *Shigella/Escherichia* |
| 52 | Proteobacteria | | |
| 53 | Tenericutes | Mollicutes | *Mycoplasma* |
| 54 | Verrucomicrobia | Verrucomicrobiae | *Akkermansia* |

Data pre-processing

To ensure high quality assurance, several quality control criteria were applied to the detection data for each sample: 1) a bead count >2 for each probe; 2) the hybridisation control (HYC) median signal >13,000; 3) a median background signal <500; and 4) a universal control median signal >4500. Normalisation was applied by first dividing the signal intensity of each probe in each sample by the signal intensity for HYC for that sample, and multiplying by 1000. This was done to adjust for sample differences due to pipetting or hybridisation. Subsequently, normalisation to adjust for run differences was applied by dividing the HYC-normalised signal of each probe in each sample by the median HYC-normalised signal of each probe for replicates of a synthetic DNA control, and multiplying by 1000. Prior to normobiotic microbiota profile calibration, normalised signal intensities below 15 were set to 0 to remove for low background noise and data was mean centred. Test and validation samples were normalised, and normalised signals below 15 were set to 0 before data was mean centred using mean probe signals from the normobiotic reference cohort.

Dysbiosis test development and validation

Principal component analysis (PCA) was used to build a normobiotic microbiota profile (model). The boundary between non-dysbiotic and dysbiotic was determined by calculating confidence regions for the values of Hotelling's T-square and Q statistics given by PCA scores in the model. Geometrically this corresponds to a rectangle with one corner located at the origin which classifies samples located within the rectangle as non-dysbiotic and samples located outside as dysbiotic. Analysis of T-square and Q statistics scaled by the confidence limit showed that the Euclidian distance from the origin had a log-normal like distribution (data not shown). Euclidian distance from the origin was used to merge the two dimensions, and weighting was performed to capture the effect of T-squared and Q statistics as appropriate. A single numeric representation of the degree of dysbiosis, defined as the Dysbiosis Index (DI), was derived from a log-normal distribution by assigning estimated portions of the distribution to different values on a scale set from 0-5. A DI value of 2 was defined as class separation represented by the identified confidence limits; a DI of 2 or lower being the non-dysbiotic region and a DI of 3 or higher being the dysbiotic region. The higher the DI above 2, the more the sample is considered to deviate from normobiosis, e.g. sample A with DI = 4 is farther away from the normobiotic reference cohort in the Euclidian space than sample B with DI = 3, thus A is more dysbiotic than B. The scale was optimised with emphasis on reducing technical variation between replicates, meaning that the integer part of the numeric output is decided by predetermined levels of the Euclidian distance.
To create the present test, 211 normal individuals were selected and randomly split into a training set (n = 165) designed to build models and a test set (n = 46) designed to tune parameters. Duplicate samples were run, and mean normalised signal was used for training and testing. Sample demographics for the two groups were similar (Table 3). Additionally, a set of IBS patients were included in the test set (*n* = 127). A number of models were developed and evaluated, and the frequency of dysbiosis in the test set was used as measure of model performance. For the final PCA model, 15 principal components were used, and a 98% confidence limit was determined for T-squared and Q statistics to define class separation. When the model is used to score other samples, values outside these limits are defined as dysbiotic.

**Table 3. Sample sets used for test development and validation**

| Cohort | Samples, *n* | Age, mean | Female, % | Sample type, n | | |
| --- | --- | --- | --- | --- | --- | --- |
| | | | | Normal | IBS | IBD |
| Training | 165 | 42 | 64 | 165 | - | - |
| Test | 173 | 40 | 73 | 46 | 127 | - |
| Validation | 287 | 39 | 71 | 43 | 109 | 135 |
| Full cohort | 625 | 40 | 70 | 254 | 236 | 135 |

External validation using an independent test set comprising normal, IBS and IBD subjects (*n* = 287) was used to assess the clinical diagnostic performance of the model (Table 4). The validation set subjects were all from unique donors who had not been included in the normal reference population used for normobiotic profile calibration or in parameter tuning. Each sample was processed using the finalised algorithm which converts data for each sample into a single integer, i.e. the DI, which represents the degree of dysbiosis based on bacterial abundance and profile within a sample relative to the established normobiotic profile. A DI > 2 represents a potentially clinically relevant deviation in microbiotic profile from that of the normobiotic reference population. Finally, the dysbiosis frequency was calculated. Additionally, PCA was performed on the validation set to investigate differences in microbiota profile between the three subject groups.

**Table 4. Percentage dysbiosis and mean DI score in validation cohort**

| Cohort | Total | Dysbiotic, % (95% CI) | DI, mean |
| --- | --- | --- | --- |
| Normal controls | 43 | 16 ($\pm$11) | 1.72 |
| IBS | 109 | 73 ($\pm$8) | 2.98 |
| IBS-D | 34 | 76 ($\pm$14) | 3.03 |
| IBS-C | 26 | 73 ($\pm$17) | 3.00 |
| IBS-M | 3 | 67 | 3.33 |
| IBS-U | 25 | 72 ($\pm$18) | 3.04 |
| IBS-A | 20 | 70 ($\pm$20) | 2.85 |
| IBD treatment-naive | 80 | 70 ($\pm$10) | 3.31 |
| CD | 30 | 80 ($\pm$14) | 3.60 |
| UC | 50 | 64 ($\pm$13) | 3.14 |
| IBD remission | 55 | 80 ($\pm$11) | 3.15 |
| CD | 18 | 89 ($\pm$14) | 3.65 |
| UC | 36 | 75 ($\pm$14) | 2.92 |

A, alternating; C, constipation; CD, Crohn's disease; D, diarrhoea; LI, Dysbiosis Index; IBD, inflammatory bowel disease; IBS, irritable bowel syndrome; M, mixed; U, un-subtyped; UC, ulcerative colitis.

Technical performance

The EU directive for in vitro diagnostic tests was followed to ensure compliance with a CE-marked test. The main technical parameters evaluated were precision and quantitative range of the test; both at probe signal level and at final output level (i.e., DI). At probe level, precision of signals (coefficient of variation [CV], percentage) varied with raw signal intensity. Signals below 500 IU were regarded as background noise; therefore measurement of variance was not applicable. For signals above 500 IU precision was estimated to be 8.4%, using repeated runs for six donors over six faecal extractions per donor over 2 days (n = 328). A CV below 10% was set as a criterion in development of the DI algorithm. Based on repetitive measurements of 139 dysbiotic samples, 94% of the samples showed CVs below 10%. In addition, several in-process test steps were evaluated (data not shown).

Faecal microbiota variation over time

Variation in microbiota over time was investigated both for normalised data across the selected probe-set, and for the test result (DI). Faecal samples were collected from five donors (aged 24-38; 80% women) at a 1-week interval for up to 14 weeks. PCA of normalised data was performed, and statistical assessment of variation in the signals for donor and sampling time (weekly) was conducted using R package *ffmanova,* an implementation of fifty-fifty multivariate analysis of variance (ANOVA).

Statistical analysis

All data were analysed at GA (Genetic Analysis AS, Oslo, Norway). Categorical data were expressed as the number of subjects (and percentage) with a specified condition or clinical variable, and the mean as appropriate. The Mann-Whitney U test was used for testing DI values. All tests were two-sided, and the chosen level of significance was $P < 0.05$. Analysis was done using the statistical computing language R version 3.0.2 and MATLAB 2011b, The Math-Works, Inc., Natick, Massachusetts, United States.

**Results**

Frequency of dysbiosis in normal, IBS and IBD subjects

Validation of the prsently described test was performed by comparing frequency of dysbiosis in a set of 287 samples, including normal individuals previously not included in the normobiotic profile calibration (n = 43) and patients with IBS (n = 109) and IBD (n = 135) (Table 3). The results in the validation cohort are given in Table 4. Of the 43 normal samples included in the validation cohort, seven (16%) were determined as being dysbiotic, with the distribution of DI scores for validation cohort shown in Figure 1. Among the IBS patients, 80 out of 109 (73%) were determined as being dysbiotic. In the IBD cohort, 100 out of 135 (74%) were determined as being dysbiotic, including 56 out of 80 (70%) treatment-naïve IBD patients, and 44 out of 55 (80%) IBD patients in clinical remission. The distribution of DI between IBS and IBD patients was significantly different (P < 0.01). Figure 1 suggests that the distribution of DI scores for the IBD cohort shows a greater shift towards higher values than IBS. Similarly, within both IBD cohorts, the frequency of dysbiosis for CD (80% and 89%, respectively) was higher than that for UC (64% and 75%), with a significant difference in DI values between CD and UC (P = 0.03).

The test was also applied to a set of 43 available samples from normal individuals from Denmark (n = 19; aged 23-61; 63% women) and Spain (n = 24; aged 22-56; 50% women). Seven of the 19 Danish samples were determined as being dysbiotic with mean DI of 2.16, resulting in 37% dysbiotic (95% CI, 15%-59%). Among the Spanish samples, 10 out of 24 were determined as being dysbiotic with mean DI of 2.58, resulting in 42% dysbiotic (95% confidence interval [CI], 22%-62%). While results for the Danish normal cohort were not significantly different from the normal validation cohort (*P* > 0.05), we observed that 50% (5/10) of the dysbiotic samples in the Spanish cohort showed a DI above 3.

Bacterial profile in dysbiosis

Applying PCA to the validation cohort using normalised data for all 54 probes demonstrated relative clustering of samples by disease cohorts. The scores for the first two principal components (PC), accounting for 48% of the variance in the data, showed a tighter cluster for normal subjects in the bottom right corner compared with a more diverse spread for subjects with IBD and IBS (Figure 2A). The sample distribution in the scores plot was found to be linked to the degree of dysbiosis, with a central cluster of non-dysbiotic samples surrounded by samples with 'weak' dysbiosis (DI = 3), and the samples with the most 'severe' dysbiosis (DI = 5) scattered outside this cluster (Figure 2B). Both the first and second principal component each separate the normal samples from IBS and IBD samples to a certain degree. The scatter of DI values implies that different bacteria dominate dysbiosis for different samples. To further investigate which bacterial groups were the main contributors to dysbiosis in IBD and IBS, differences in overall mean normalised signal between dysbiotic and non-dysbiotic status for each of the 54 probes were calculated. The predominant bacteria contributing to dysbiosis within the IBS cohort were Firmicutes (Bacilli), Proteobacteria (*Shigella*/*Escherichia*), Actinobacteria and *Ruminococcus gnavus* (Figure 3A). Similarly, the predominant bacteria within the IBD cohort were Proteobacteria (*Shigella*/*Escherichia*), Firmicutes, specifically *Faecalibacterium prausnitzii,* and Bacteroidetes (Bacteroides and Prevotella) (Figure 3B). Interestingly, Proteobacteria *(Shigella*/*Escherichia)* was among the top five dysbiosis-contributing bacterial groups for both IBS and IBD, implying similarities in dysbiosis between IBS and IBD. However, all bacterial groups that contributed most to dysbiosis in the IBS cohort showed increased probe signal intensity compared to non-dysbiotic patients, while for the IBD cohort, both reduced (*F. prausnitzii*) and increased probe signal intensities were the main contributors to dysbiosis.

We found a single probe with a differential signal between samples from the Spanish and Scandinavian cohorts (*P* < 0.01; Benjamini-Hochberg correction). The probe targets Firmicutes (*Streptococcus*), and this signal was found to be elevated in the Spanish samples compared to the Scandinavian cohort. Figure 4 shows the predominant bacteria contributing to dysbiosis within the Spanish samples. As expected, Proteobacteria (*Shigella*/*Escherichia*) is again found to be a contributing bacteria in dysbiosis. Additionally, *Bacteroides stercoris* and *Bifidobacterium* contribute to dysbiosis, which potentially could be linked to differences in e.g. diet between Scandinavian countries and the Mediterranean region.

Faecal microbiota variation over time

Faecal samples were collected from five individuals at 1-week intervals for up to 14 weeks. PCA of the normalised data (*n* = 64) revealed that most variability in the longitudinal faecal microbiota analysis was related to inter-individual variability; donors could clearly be distinguished by the three first and most important PCs in the score plot (Figure 5). In this study, samples were clustered according to faecal donor independently of sample collection time. The three first PCs described 65% of the total variability in the faecal microbiota data.

The significance of the PCs was analysed by *ffmanova* using normalised data and only the main effects of donor and sampling time (weekly) were included in the model. The results show that the average amount of variation between donors was greater than that within a donor (*P* < 0.001) with explained variances based on sums of squares of 0.48. The variation between sampling time was not significant (*P* = 0.26), with explained variances based on sums of squares of 0.11. The low level of variation within one individual over time is crucial in utilising the test for monitoring changes during treatment for the purpose of altering the microbiota profile.

**Discussion**

In this Example, we demonstrate the performance of a novel gut microbiota test, aiming to identify and characterise dysbiosis by determining deviation from normobiosis. Such a diagnostic approach contrasts to direct diagnosis of a particular disease. Characteristic sets of bacteria are required in a healthy normobiotic gut microbiota, and deviation will represent a dysbiotic state. Quantitative measurement of deviation in bacterial microbiota makes it possible to characterise dysbiosis in samples from IBS and IBD patients based on a single diagnostic algorithm targeting normobiosis.

The present test is a broad-spectrum, reproducible, precise, high-throughput, easy to use method of quantifying the extent of dysbiosis that is especially suitable for clinical use. This test gives an algorithmically-derived DI based on bacterial abundance and profile within a sample. This DI is an indicator of the degree to which an individual's microbiome deviates from that of a normal reference population and could potentially be highly relevant in clinical diagnosis and monitoring of the progression of conditions such as IBD and IBS. The stability of the human gut microbiota is another important feature if microbial characterisation is to play a role in diagnosis, treatment, and prevention of disease. It has been shown that, in an individual's microbiota, 60% of the bacterial strains persisted over the course of 5 years. In our corresponding study, we found only a low within-individual variation in weekly sampling over 14 weeks.

The presently described test has been used to detect high frequency of dysbiosis in IBS and IBD patients and low frequency in normal individuals. Both IBD patients in remission and treatment-naïve IBD patients reported DI scores well above the threshold of 2 with a dysbiosis frequency of 80% and 70%, respectively. Rome III-diagnosed IBS patients showed a frequency of 73%, confirming previous observations, while the frequency in normal individuals was 16%.

Dysbiosis is associated with many diseases, including IBS, different forms of IBD, obesity and diabetes, and has also been implicated in depression and autism. In recent years, new treatment options have emerged with respect to restoring the balance of the microbiota in dysbiotic patients. FMT is now regarded as the most effective treatment in relapsing *Clostridium difficile* colitis and is currently being studied in phase I to IV clinical trials in many of the aforementioned conditions (CD, phase II/III NCT01793831; UC, phase I NCT01947101, phase II NCT01896635, phase II/III NCT01790061; IBD including CD and UC, phase IV NCT02033408).

A key barrier in the interpretation of FMT data has been the variability in bacterial composition of donor microbiota, not only related to pathogenic organisms but also to the composition of the normally occurring microflora, further highlighting the importance of identifying a method to sufficiently characterise both pathogenic and non-pathogenic microbes. The ability to characterise an individual's microbiome and monitor alterations may allow for the prediction of therapeutic outcome or even relapse in such conditions. It may also help to explain why a patient is refractory to particular therapeutic regimens and aid adaptation of the regimen accordingly. Furthermore, rapid and reproducible detailed bacterial profiles from normobiotic and dysbiotic individuals may aid the continuation of innovative therapeutic approaches such as FMT. Thus, use of the test could prove clinically useful in determining dysbiosis, not only in IBS and IBD patients, but also in other conditions where knowledge about the microbiota profile might prove clinically useful, in the subsequent monitoring of prescribed treatment regimens, and in the evolution of new therapeutic approaches.

**EXAMPLE 2** - Representative analysis of a sample using 54 probes targeting a plurality of microorganisms or groups of microorganisms

**Table 5: Measured signals for 54 test probes and 4 control probes (bold)**

| Probe | 0 | 1 | 2 | 3 | 4 | 5 | 6 | 7 | 8 | 9 |
|---|---|---|---|---|---|---|---|---|---|---|
| Measured signal | 1187 | 41 | 4411 | 2198 | 9 | 10 | 89 | 38 | 50 | 1691 |
| Probe | 10 | 11 | 12 | 13 | 14 | 15 | 16 | 17 | 18 | 19 |
| Measured signal | 1358 | 27 | 60 | 303 | 4330 | 26 | 26 | 250 | 44 | 885 |
| Probe | 20 | 21 | 22 | 23 | 24 | 25 | 26 | 27 | 28 | 29 |
| Measured signal | 1369 | 7 | 68 | 65 | 49 | 24 | 10 | 32 | 45 | 67 |
| Probe | 30 | 31 | 32 | 33 | 34 | 35 | 36 | 37 | 38 | 39 |
| Measured signal | 2676 | 798 | 17 | 2 | 2 | 528 | 32 | 203 | 1 | 529 |
| Probe | 40 | 41 | 42 | 43 | 44 | 45 | 46 | 47 | 48 | 49 |
| Measured signal | 765 | 38 | 97 | 183 | 120 | 8 | 5068 | 4548 | 4 | 47 |
| Probe | 50 | 103 | 104 | 105 | **106** | **107** | **126** | **127** | | |
| Measured signal | 1 | 155 | 419 | 11 | **81** | **19.352** | **4** | **6** | | |

**Table 6: Measured signals adjusted by hybridisation control probe (107) - test values divided by value of 107 probe.**

| Probe | 0 | 1 | 2 | 3 | 4 | 5 | 6 | 7 | 8 | 9 |
|---|---|---|---|---|---|---|---|---|---|---|
| Hyb adjusted data | 61.33732947 | 2.118644068 | 227.9350971 | 113.5799917 | 0.46506821 | 0.516742456 | 4.599007854 | 1.963621331 | 2.583712278 | 87.38114924 |
| Probe | 10 | 11 | 12 | 13 | 14 | 15 | 16 | 17 | 18 | 19 |
| Hyb adjusted data | 70.17362547 | 1.39520463 | 3.100454733 | 15.6572964 | 223.7494833 | 1.343530384 | 1.343530384 | 12.91856139 | 2.273666804 | 45.73170732 |
| Probe | 20 | 21 | 22 | 23 | 24 | 25 | 26 | 27 | 28 | 29 |
| Hyb adjusted data | 70.74204217 | 0.361719719 | 3.513848698 | 3.358825961 | 2.532038032 | 1.240181893 | 0.516742456 | 1.653575858 | 2.32534105 | 3.462174452 |
| Probe | 30 | 31 | 32 | 33 | 34 | 35 | 36 | 37 | 38 | 39 |
| Hyb adjusted data | 138.2802811 | 41.23604795 | 0.878462174 | 0.103348491 | 0.103348491 | 27.28400165 | 1.653575858 | 10.48987185 | 0.051674246 | 27.3356759 |
| Probe | 40 | 41 | 42 | 43 | 44 | 45 | 46 | 47 | 48 | 49 |
| Hyb adjusted data | 39.53079785 | 1.963621331 | 5.012401819 | 9.456386937 | 6.200909467 | 0.413393964 | 261.8850765 | 235.0144688 | 0.206696982 | 2.428689541 |
| Probe | 50 | 103 | 104 | 105 | | | | | | |
| Hyb adjusted data | 0.051674246 | 8.009508061 | 21.65150889 | 0.568416701 | | | | | | |

EP 3 274 885 B1

**Table 7: Data set following normalisation**

| Probe | 0 | 1 | 2 | 3 | 4 | 5 | 6 | 7 | 8 | 9 |
|---|---|---|---|---|---|---|---|---|---|---|
| Normalised data | 39.90146827 | 2.049095604 | 226.4348805 | 110.1761624 | 1.053325259 | 0.434377731 | 5.013782072 | 1.776521563 | 4.335974759 | 55.75664619 |
| Probe | 10 | 11 | 12 | 13 | 14 | 15 | 16 | 17 | 18 | 19 |
| Normalised data | 46.20320406 | 1.067156835 | 2.956232674 | 14.28966269 | 164.7199777 | 1.69062125 | 1.752930721 | 12.71695229 | 1.494773273 | 38.19827022 |
| Probe | 20 | 21 | 22 | 23 | 24 | 25 | 26 | 27 | 28 | 29 |
| Normalised data | 45.59413493 | 0.302402828 | 2.96922674 | 3.293599131 | 2.274834376 | 0.996537623 | 0.346558113 | 1.066689323 | 1.431661161 | 2.368829251 |
| Probe | 30 | 31 | 32 | 33 | 34 | 35 | 36 | 37 | 38 | 39 |
| Normalised data | 97.0767168 | 32.21356304 | 1.70558488 | 0.086343829 | 0.186862151 | 19.53707819 | 1.531364753 | 6.975498538 | 0.103842051 | 26.25166552 |
| Probe | 40 | 41 | 42 | 43 | 44 | 45 | 46 | 47 | 48 | 49 |
| Normalised data | 33.57748097 | 2.117601983 | 2.816319044 | 7.686220382 | 4.397992441 | 0.40089312 | 214.4184064 | 230.71127 | 0.131890189 | 2.232690034 |
| Probe | 50 | 103 | 104 | 105 | | | | | | |
| Normalised data | 0.038252614 | 7.013373509 | 27.69737264 | 0.60197028 | | | | | | |

EP 3 274 885 B1

**Table 8: Data set checked for background**

| Probe | 0 | 1 | 2 | 3 | 4 | 5 | 6 | 7 | 8 | 9 |
|---|---|---|---|---|---|---|---|---|---|---|
| BG checked data | 39.90146827 | 0 | 226.4348805 | 110.1761624 | 0 | 0 | 0 | 0 | 0 | 55.75664619 |
| Probe | 10 | 11 | 12 | 13 | 14 | 15 | 16 | 17 | 18 | 19 |
| BG checked data | 46.20320406 | 0 | 0 | 0 | 164.7199777 | 0 | 0 | 0 | 0 | 38.19827022 |
| Probe | 20 | 21 | 22 | 23 | 24 | 25 | 26 | 27 | 28 | 29 |
| BG checked data | 45.59413493 | 0 | 0 | 0 | 0 | 0 | 0 | 0 | 0 | 0 |
| Probe | 30 | 31 | 32 | 33 | 34 | 35 | 36 | 37 | 38 | 39 |
| BG checked data | 97.0767168 | 32.21356304 | 0 | 0 | 0 | 19.53707819 | 0 | 0 | 0 | 26.25166552 |
| Probe | 40 | 41 | 42 | 43 | 44 | 45 | 46 | 47 | 48 | 49 |
| BG checked data | 33.57748097 | 0 | 0 | 0 | 0 | 0 | 214.4184064 | 230.71127 | 0 | 0 |
| Probe | 50 | 103 | 104 | 105 | | | | | | |
| BG checked data | 0 | 0 | 27.69737264 | 0 | | | | | | |

**Table 9: Data set following centering**

| Probe | 0 | 1 | 2 | 3 | 4 | 5 | 6 | 7 | 8 | 9 |
|---|---|---|---|---|---|---|---|---|---|---|
| Centred data | -27.02354384 | -12.62947631 | -28.507661 | -44.08041189 | -40.59134184 | -12.27079637 | -21.76437749 | -46.35334588 | -73.46749748 | -51.51981803 |
| Probe | 10 | 11 | 12 | 13 | 14 | 15 | 16 | 17 | 18 | 19 |
| Centred data | -301.3688994 | -17.21722068 | -8.046376162 | -56.99541858 | -39.70909832 | -44.67724211 | -0.926414605 | -38.19314123 | -154.2626382 | -22.98898606 |
| Probe | 20 | 21 | 22 | 23 | 24 | 25 | 26 | 27 | 28 | 29 |
| Centred data | -43.06202196 | -119.7774288 | -71.12408019 | -40.62803664 | -569.8209942 | -1.989581656 | -1.713457687 | -87.14426606 | -0.100282038 | -67.94087802 |
| Probe | 30 | 31 | 32 | 33 | 34 | 35 | 36 | 37 | 38 | 39 |
| Centred data | -49.52345234 | -83.87950772 | -2.08008984 | 0 | -1.989008551 | -27.44811268 | -39.2686822 | -171.9883911 | -37.67136638 | -11.79766251 |
| Probe | 40 | 41 | 42 | 43 | 44 | 45 | 46 | 47 | 48 | 49 |
| Centred data | -142.341303 | -24.07898983 | -645.760442 | -152.3347191 | -328.003766 | -40.9444343 | -177.9405193 | -327.5029234 | -195.1030625 | -2.329866583 |
| Probe | 50 | 103 | 104 | 105 | | | | | | |
| Centred data | -36.92478523 | -40.85323644 | -31.56030435 | -8.717386493 | | | | | | |

**Table 10: Data set projected by 15 loading vector (Step (ii))**

| Loading vector | PC1 | PC2 | PC3 | PC4 | PC5 | PC6 | PC7 | PC8 |
|---|---|---|---|---|---|---|---|---|
| Data projected by loading vector | 619.2184977 | -402.8737744 | -207.5503637 | 91.30908749 | 183.3507408 | -77.14217491 | 543.9111741 | -142.5242892 |
| Loading vector | PC9 | PC10 | PC11 | PC12 | PC13 | PC14 | PC15 | |
| Data projected by loading vector | 489.361778 | 172.0068772 | -137.6666566 | 0.432754519 | -113.8208536 | -104.5873989 | -40.45465493 | |

**Table 11: Reinflated data set (Step (iii))**

| Probe | 0 | 1 | 2 | 3 | 4 | 5 | 6 | 7 | 8 | 9 |
|---|---|---|---|---|---|---|---|---|---|---|
| Reinflated data | -16.28527981 | 4.282976317 | -21.0697846 | -11.87483876 | -30.26649115 | 5.141867717 | -2.597888749 | -41.2071567 | -105.8671823 | -110.2352987 |
| Probe | 10 | 11 | 12 | 13 | 14 | 15 | 16 | 17 | 18 | 19 |
| Reinflated data | -271.7114099 | -47.26707379 | -13.36384232 | -3.708870897 | -23.22096946 | -35.53919007 | -5.15748711 | -4.006900436 | -167.1165208 | -22.55549691 |
| Probe | 20 | 21 | 22 | 23 | 24 | 25 | 26 | 27 | 28 | 29 |
| Reinflated data | -59.84469431 | -147.4498659 | -80.69592993 | -42.95781022 | -591.2771802 | -16.57525981 | -6.161210071 | -60.45008896 | -1.600920004 | -79.04167189 |
| Probe | 30 | 31 | 32 | 33 | 34 | 35 | 36 | 37 | 38 | 39 |
| Reinflated data | -68.74911311 | -110.6602281 | -4.444441798 | 2.678325 | 1.082427477 | 0.27543201 | -70.5725879 | -177.80101 | -60.90536079 | -7.821457405 |
| Probe | 40 | 41 | 42 | 43 | 44 | 45 | 46 | 47 | 48 | 49 |
| Reinflated data | -153.4686911 | -20.35932606 | -629.1116591 | -133.4222904 | -314.7722453 | -42.34584901 | -102.0087072 | -321.0313364 | -163.2957603 | -3.59112869 |
| Probe | 50 | 103 | 104 | 105 | | | | | | |
| Reinflated data | -39.71174924 | -35.69931905 | -12.62559789 | -23.73199124 | | | | | | |

**Table 12: Square of difference between centered data (Table 9) and reinflated data set (Table 11) (Step (iv))**

| Probe | 0 | 1 | 2 | 3 | 4 | 5 | 6 | 7 | 8 | 9 |
|---|---|---|---|---|---|---|---|---|---|---|
| Difference | 10.73826403 | 16.91245262 | 7.437876402 | 32.20557313 | 10.32485069 | 17.41266409 | 19.16648874 | 5.146189184 | -32.39968487 | -58.71548071 |
| Square | 115.3103144 | 286.0310537 | 55.32200537 | 1037.19894 | 106.6025418 | 303.2008705 | 367.3542906 | 26.48326312 | 1049.73958 | 3447.507675 |
| Probe | 10 | 11 | 12 | 13 | 14 | 15 | 16 | 17 | 18 | 19 |
| Difference | 29.65748947 | -30.04985311 | -5.31746616 | 53.28654769 | 16.48812886 | 9.138052039 | -4.231072504 | 34.18624079 | -12.85388267 | 0.433489153 |
| Square | 879.5666819 | 902.9936719 | 28.27544636 | 2839.456164 | 271.8583934 | 83.50399506 | 17.90197454 | 1168.699059 | 165.2222997 | 0.187912846 |
| Probe | 20 | 21 | 22 | 23 | 24 | 25 | 26 | 27 | 28 | 29 |
| Difference | -16.78267235 | -27.67243716 | -9.57184974 | -2.329773577 | -21.45618596 | -14.58567815 | -4.447752384 | 26.69417709 | -1.500637966 | -11.10079386 |
| Square | 281.6580911 | 765.7637781 | 91.62030744 | 5.427844921 | 460.3679159 | 212.7420072 | 19.78250127 | 712.5790907 | 2.251914306 | 123.2276244 |
| Probe | 30 | 31 | 32 | 33 | 34 | 35 | 36 | 37 | 38 | 39 |
| Difference | -19.22566077 | -26.78072039 | -2.364351958 | 2.67833E-22 | 3.071436028 | 27.72354469 | -31.30390571 | -5.812618944 | -23.23399441 | 3.976205105 |
| Square | 369.6260321 | 717.2069848 | 5.590160183 | 7.17343E-44 | 9.433719272 | 768.5949302 | 979.9345125 | 33.78653898 | 539.8184964 | 15.81020704 |
| Probe | 40 | 41 | 42 | 43 | 44 | 45 | 46 | 47 | 48 | 49 |
| Difference | -11.12738806 | 3.719663769 | 16.64878298 | 18.9124287 | 13.23152074 | -1.401414709 | 75.93181214 | 6.471586973 | 31.80730217 | -1.261262107 |
| Square | 123.818765 | 13.83589856 | 277.1819746 | 357.6799594 | 175.0731412 | 1.963963185 | 5765.640095 | 41.88143795 | 1011.704471 | 1.590782101 |
| Probe | 50 | 103 | 104 | 105 | | | | | | |
| Difference | -2.786964008 | 5.153917393 | 18.93470646 | -15.01460475 | | | | | | |
| Square | 7.767168384 | 26.56286449 | 358.5231088 | 225.4383557 | | | | | | |

Sum of squared differences (Qres): 27656.3

Sum of values after eigenvalues applied to projected data set (Table 10): 77.30951 (Hotelling's $T^2$; step (v))

Qres and Hotelling's $T^2$ values were then compared to predetermined normobiotic to dysbiotic threshold values: [$T^2$ = 32.49 and Qres = 42834.81]. Dysbiosis was confirmed as likely as $T^2$ value exceeds threshold.

$T^2$ and Qres were then combined into a single metric using squares; weights (0.938 and 0.157) and square root (i.e. Formula III). The resulting figure was 2.318146

The resulting single metric was then plotted on numerical scale with a normobiotic to dysbiotic class separation point of 0.395 (representative value 2), and further thresholds at 1.632 (representative value 3), 2.492 (representative value 4) and infinity at a representative value of 5. This placed the sample between thresholds represented by the values 3 and 4 (close to the upper limit of 2.492 on the interval from 3 to 4). The precise location of the sample of this scale was then calculated as follows:

Total log normal distribution density area between 3 and 4 was calculated:

0.6820813
0.4840499
0.1980315
(first minus second equals third)

The log normal distribution density area between 3 and the sample was then calculated:

0.6502034
0.4840499
0.1661535
(first minus second equals third)

The log normal distribution density area between 3 and the sample was then divided by the log normal distribution density area between 3 and 4 to find the precise fraction: 0.8390257. The lower integer (3) was then added to get the precise position on the scale: 3.839026. This was then rounded up to 4 for reporting.

## Claims

1. A computer-implemented method for determining the likelihood of GI tract dysbiosis in a subject, said method comprising:

   (i) providing a test data set, wherein said test data set comprises at least one microbiota profile, said microbiota profile being a profile of the relative levels of a plurality of microorganisms or groups of microorganisms in a sample from the GI tract of the subject and wherein each level of each microorganism or group of microorganisms is a profile element of said test data set,
   (ii) applying to said test data set at least one loading vector, wherein said at least one loading vector is at least one orthogonal latent variable determined by the regression analysis of the levels of said plurality of microorganisms or groups of microorganisms in corresponding GI tract samples from a plurality of normal subjects, thereby producing a first projected data set,
   (iii) providing said first projected data set,
   (iv) from said first projected data set calculating the Q-residual of the microbiota profile and comparing the Q-residual of the microbiota profile with a normobiotic to dysbiotic threshold Q-residual value determined from the corresponding analysis of said plurality of microorganisms or groups of microorganisms in corresponding GI tract samples from a plurality of normal subjects and/or subjects with dysbiosis,
   (v) from said first projected data set calculating the Hotelling's $T^2$ for the microbiota profile from the variance explained by the orthogonal latent variables determined by the regression analysis of the levels of said plurality of microorganisms or groups of microorganisms in corresponding GI tract samples from a plurality of normal subjects of step (ii) and comparing said Hotelling's $T^2$ for the microbiota profile with a normobiotic to dysbiotic threshold Hotelling's $T^2$ value determined from the corresponding analysis of said plurality of microorganisms or groups of microorganisms in corresponding GI tract samples from a plurality of normal subjects and/or a plurality of subjects with dysbiosis,

   wherein step (v) may be performed before or after or concurrently with step (iv), and wherein a microbiota profile with a Q-residual or Hotelling's $T^2$ in excess of said respective thresholds is indicative of a likelihood of dysbiosis.

2. The computer-implemented method of claim 1 wherein said test data set comprises a plurality of microbiota profiles and said test data set is arranged into a matrix.

3. The computer-implemented method of claim 1 or claim 2, wherein said orthogonal latent variables are determined by the orthogonal transformation into principal components of the levels of said plurality of microorganisms or groups of microorganisms in corresponding GI tract samples from a plurality of normal subjects, preferably wherein the orthogonal transformation into principal components is by at least one of partial least squares regression analysis, Principal Component Analysis, canonical correlation analysis, redundancy analysis, correspondence analysis, and canonical correspondence analysis.

4. The computer-implemented method of any preceding claim, wherein at least 2 loading vectors, preferably at least 3, 5, 7, 9, 11, 13, 15, 17, 19 or 20 loading vectors, and/or no more than 50 loading vectors, preferably no more than 40, 30, 25, 20, or 15 loading vectors are applied.

5. The computer-implemented method of any preceding claim, wherein the loading vector is applied in the form of a projection matrix.

6. The computer-implemented method of any preceding claim, wherein said microbiota profiles are quantitative or semi-quantitative and wherein said method provides a quantitative or semi-quantitative measure of the extent of dysbiosis.

7. A computer-implemented method for quantifying dysbiosis, said method comprising performing the method of claim 6, wherein said comparisons with normobiotic to dysbiotic thresholds together comprise combining the Q-residual of step (iv) and the Hotelling's $T^2$ of step (v) into a single metric for dysbiosis, preferably wherein the Euclidean distance from the origin for both the Q-residual of step (iv) and the Hotelling's $T^2$ of step (v) is calculated and preferably wherein the Euclidean distance from the origin for both Q-residual and Hotelling's $T^2$ is calculated with Formula I:

$$r = \sqrt{\{T^2\}^2 + Qres^2}$$

8. The computer-implemented method of claim 7 wherein the combining of the Q-residual of step (iv) and the Hotelling's $T^2$ of step (v) into a single metric for dysbiosis comprises scaling said Q-residual of step (iv) and Hotelling's $T^2$ of step (v) to result in values of similar magnitude.

9. The computer-implemented method of claim 7 or claim 8, wherein said single metric is plotted on a finite numerical scale with a normobiosis to dysbiosis class separation at a predetermined point on said finite numerical scale which represents, or is, a combination of the normobiotic to dysbiotic class thresholds of steps (iv) and (v), similarly scaled if scaling has been applied.

10. The computer-implemented method of claim 7 or claim 8, wherein said single metric is plotted on a finite numerical scale with a normobiosis to dysbiosis class separation at a predetermined point on said finite numerical scale, and wherein

(a) for a test sample having at least one of the Q-residual of step (iv) or Hotelling's $T^2$ of step (v) above the normobiotic to dysbiotic class threshold values of steps (iv) and (v), respectively, said class separation point corresponds to that of one or other of the exceeded normobiotic to dysbiotic class threshold value of steps (iv) or (v), similarly scaled if scaling has been applied, and
(b) for a test sample in which neither of the Q-residual of step (iv) or the Hotelling's $T^2$ of step (v) are beyond the normobiotic to dysbiotic threshold values of steps (iv) and (v), respectively, said class separation point corresponds to the sum of the normobiotic to dysbiotic class thresholds of steps (iv) and (v), similarly scaled if scaling has been applied.

11. The computer-implemented method of any one of claims 7 to10, wherein weightings are applied to the Q-residual of step (iv) and the Hotelling's $T^2$ of step (v) during the second combination step, and wherein said weightings minimise the effects of technical variation.

12. A computer-implemented method for diagnosing, monitoring and/or characterising diseases and conditions associated with perturbations in the microbiota of the GI tract or the assessing of the risk of developing a disease or condition which is associated with a perturbation of the microbiota profile of the GI tract, said method comprising performing a method as defined in any one of claims 1 to 11, wherein the indication the likelihood of dysbiosis or the extent of dysbiosis is indicative of the presence or absence, the risk of developing, the progress of, or the characteristics of said disease or condition associated with perturbations in the microbiota of the GI tract.

13. The computer-implemented method of claim 12 wherein said disease or condition associated with a perturbation in the microbiota of the GI tract is selected from functional GI tract disorders, small bowel bacterial overgrowth syndrome, GI tract cancers, breast cancer, ankylosing spondylitis; non-alcoholic steatohepatitis; atopic diseases, metabolic disorders, neurological disorders, autoimmune diseases, malnutrition, chronic fatigue syndrome and autism.

14. The computer-implemented method of any preceding claim, wherein said method further comprises a preceding step in which at least one of said microbiota profiles is prepared, preferably wherein said step of preparing said microbiota profiles comprises nucleic acid analysis, preferably nucleic acid sequencing, oligonucleotide probe hybridisation, primer based nucleic acid amplification; antibody or other specific affinity ligand based detection; proteomic analysis or metabolomic analysis, and preferably wherein the sample from the GI tract is selected from

(a) luminal contents of the GI tract, preferably stomach contents, intestinal contents, mucus and faeces/stool, or combinations thereof,
(b) parts of the mucosa, the submucosa, the muscularis externa, the adventitia and/or the serosa of a GI tract tissue/organ,
(c) nucleic acid prepared from (a) or (b), preferably by reverse transcription and/or nucleic acid amplification, or
(d) a microbial culture of (a) or (b).

15. A computer, system or apparatus carrying a program adapted to perform the method of any preceding claim, preferably wherein said system or apparatus is further adapted to perform microbiota profiling or a step thereof.

**Patentansprüche**

1. Computerimplementiertes Verfahren zum Bestimmen der Wahrscheinlichkeit einer GI-Trakt-Dysbiose bei einem Subjekt, wobei das Verfahren umfasst:

(i) Bereitstellen eines Testdatensatzes, wobei der Testdatensatz mindestens ein Mikrobiota-Profil umfasst, wobei das Mikrobiota-Profil ein Profil der relativen Mengen einer Vielzahl von Mikroorganismen oder Gruppen von Mikroorganismen in einer Probe aus dem GI-Trakt des Subjekts ist und wobei jede Menge jedes Mikroorganismus oder jeder Gruppe von Mikroorganismen ein Profilelement des Testdatensatzes ist,
(ii) Anwenden mindestens eines Ladevektors auf den Testdatensatz, wobei der mindestens eine Ladevektor mindestens eine orthogonale latente Variable ist, die durch die Regressionsanalyse der Mengen der Vielzahl von Mikroorganismen oder Gruppen von Mikroorganismen in dem entsprechenden GI-Trakt-Proben aus einer Vielzahl von normalen Subjekten bestimmt wird, wodurch ein erster projizierter Datensatz erzeugt wird,
(iii) Bereitstellen des ersten projizierten Datensatzes,
(iv) aus dem ersten projizierten Datensatz, Berechnen des Q-Rests des Mikrobiota-Profils und Vergleichen des Q-Rests des Mikrobiota-Profils mit einem normobiotischen bis dysbiotischen Schwellenwert-Q-Restwerts, der aus der entsprechenden Analyse der Vielzahl von Mikroorganismen oder Gruppen von Mikroorganismen in entsprechenden GI-Trakt-Proben von einer Vielzahl von normalen Subjekten und/oder Subjekten mit Dysbiose bestimmt wurde,
(v) aus dem ersten projizierten Datensatz, Berechnen von Hotelling-$T^2$ für das Mikrobiota-Profil aus der Varianz, die durch die orthogonalen latenten Variablen erklärt wird, die durch die Regressionsanalyse der Mengen der Vielzahl von Mikroorganismen oder Gruppen von Mikroorganismen in entsprechenden GI-Trakt-Proben aus einer Vielzahl von normalen Probanden von Schritt (ii) bestimmt wurden und Vergleichen des Hotelling-$T^2$ für das Mikrobiota-Profil mit einem normobiotischen bis dysbiotischen Schwellenwert-Hotelling-$T^2$-Wert, der aus der entsprechenden Analyse der Vielzahl von Mikroorganismen oder Gruppen von Mikroorganismen in entsprechenden GI-Trakt-Proben aus einer Vielzahl von normalen Subjekten und/oder einer Vielzahl Subjekten mit Dysbiose bestimmt wurde,

wobei Schritt (v) vor oder nach oder gleichzeitig mit Schritt (iv) durchgeführt werden kann und wobei ein Mikrobiota-Profil mit einem Q-Rest oder Hotelling-$T^2$, welche die jeweiligen Schwellenwerte überschreiten, ein Hinweis auf die Wahrscheinlichkeit einer Dysbiose ist.

2. Computerimplementiertes Verfahren nach Anspruch 1, wobei der Testdatensatz eine Vielzahl von Mikrobiota-Profilen umfasst und der Testdatensatz in einer Matrix angeordnet ist.

3. Computerimplementiertes Verfahren nach Anspruch 1 oder Anspruch 2, wobei die orthogonalen latenten Variablen durch die orthogonale Transformation in Hauptkomponenten der Mengen der Vielzahl von Mikroorganismen oder Gruppen von Mikroorganismen in entsprechenden GI-Trakt-Proben aus einer Vielzahl von normalen Subjekten bestimmt werden, vorzugsweise wobei die orthogonale Transformation in Hauptkomponenten durch mindestens eines von der partiellen Regressionsanalysen der kleinsten Quadrate, der Hauptkomponentenanalyse, der kanonischen Korrelationsanalyse, der Redundanzanalyse, der Korrespondenzanalyse und der kanonischen Korrespondenzanalyse erfolgt.

4. Computerimplementiertes Verfahren nach einem der vorstehenden Ansprüche, wobei mindestens 2 Ladevektoren, vorzugsweise mindestens 3, 5, 7, 9, 11, 13, 15, 17, 19 oder 20 Ladevektoren und/oder nicht mehr als 50 Ladevektoren, vorzugsweise nicht mehr als 40, 30, 25, 20 oder 15 Ladevektoren angewendet werden.

5. Computerimplementiertes Verfahren nach einem der vorstehenden Ansprüche, wobei der Ladevektor in Form einer Projektionsmatrix angewendet wird.

6. Computerimplementiertes Verfahren nach einem der vorstehenden Ansprüche, wobei die Mikrobiota-Profile quantitativ oder semi-quantitativ sind und wobei das Verfahren ein quantitatives oder semi-quantitatives Maß für das Ausmaß der Dysbiose bereitstellt.

7. Computerimplementiertes Verfahren zur Quantifizierung der Dysbiose, wobei das Verfahren das Durchführen des Verfahrens nach Anspruch 6 umfasst, wobei die Vergleiche mit normobiotischen zu dysbiotischen Schwellenwerten zusammen das Kombinieren des Q-Rests von Schritt (iv) und des Hotelling-$T^2$ von Schritt (v) zu einer einzigen Metrik für Dysbiose umfassen, vorzugsweise wobei der euklidische Abstand vom Ursprung sowohl für den Q-Rest von Schritt (iv) als auch für den Hotelling-$T^2$ von Schritt (v) berechnet wird und vorzugsweise wobei der euklidische Abstand vom Ursprung sowohl für den Q-Rest als auch für Hotelling-$T^2$ aus der Formel I berechnet wird:

$$r = \sqrt{\{T^2\}^2 + Qres^2}$$

8. Computerimplementiertes Verfahren nach Anspruch 7, wobei das Kombinieren des Q-Rests von Schritt (iv) und das Hotelling-$T^2$ von Schritt (v) in eine einzelne Metrik für Dysbiose das Skalieren des Q-Rests von Schritt (iv) und des Hotelling-$T^2$ von Schritt (v) umfasst, um Werte ähnlicher Größe zu ergeben.

9. Computerimplementiertes Verfahren nach Anspruch 7 oder Anspruch 8, wobei die einzelne Metrik auf einer endlichen numerischen Skala mit einer Normobiose-Dysbiose-Klassentrennung an einem vorbestimmten Punkt auf der endlichen numerischen Skala aufgetragen ist, die eine Kombination der normobiotischen bis dysbiotischen Klassenschwellenwerte von Schritt (iv) und (v) darstellt oder ist, ähnlich skaliert, als ob die Skalierung angewendet worden wäre.

10. Computerimplementiertes Verfahren nach Anspruch 7 oder Anspruch 8, wobei die einzelne Metrik auf einer endlichen numerischen Skala mit einer normobiotischen bis dysbiotischen Klassentrennung an einem vorbestimmten Punkt auf der endlichen numerischen Skala aufgetragen ist und wobei

(a) für eine Testprobe mit mindestens einem der Q-Reste von Schritt (iv) oder Hotelling-$T^2$ von Schritt (v) über den Schwellenwerten der normobiotischen bis dysbiotischen Klasse der Schritte (iv) bzw. (v) der Klassentrennpunkt dem des einen oder anderen der überschrittenen Schwellenwerte für normobiotische bis dysbiotische Klassen der Schritte (iv) oder (v) entspricht, ähnlich skaliert, als ob eine Skalierung angewendet worden wäre und
(b) für eine Testprobe, bei der weder der Q-Rest von Schritt (iv) noch der Hotelling-$T^2$ von Schritt (v) über den normobiotischen bis dysbiotischen Schwellenwerten der Schritte (iv) bzw. (v) liegen, wobei der Klassentrennpunkt der Summe der Schwellenwerte für normobiotische bis dysbiotische Klassenschwellenwerte der Schritte (iv) und (v) entspricht, ähnlich skaliert, als ob eine Skalierung angewendet worden wäre.

**11.** Computerimplementiertes Verfahren nach einem der Ansprüche 7 bis 10, wobei Gewichtungen auf den Q-Rest von Schritt (iv) und Hotelling-$T^2$ von Schritt (v) während des zweiten Kombinationsschritts angewendet werden und wobei diese Gewichtungen die Auswirkungen der technischen Variation minimieren.

**12.** Computerimplementiertes Verfahren zur Diagnose, Überwachung und/oder Charakterisierung von Krankheiten und Zuständen, die Störungen in der Mikrobiota des GI-Trakts zugeordnet sind, oder zur Bewertung des Risikos, eine Krankheit oder einen Zustand zu entwickeln, der einer Störung des Mikrobiota-Profils des GI-Trakts zugeordnet ist, wobei das Verfahren das Durchführen eines Verfahrens nach einem der Ansprüche 1 bis 11 umfasst, wobei die Angabe der Wahrscheinlichkeit einer Dysbiose oder des Ausmaßes der Dysbiose das Vorhandensein oder Fehlen, das Risiko der Entwicklung, den Fortschritt von oder die Charakteristika der Krankheit oder des Zustands angeben, die Störungen in der Mikrobiota des GI-Trakts zugeordnet sind.

**13.** Computerimplementiertes Verfahren nach Anspruch 12, wobei die Krankheit oder der Zustand, die einer Störung in der Mikrobiota des GI-Trakts zugeordnet sind, ausgewählt sind aus funktionellen Störungen des GI-Trakts, bakteriellem Überwuchersyndrom des Dünndarms, Krebs des GI-Trakts, Brustkrebs, Spondylitis ankylosans; nichtalkoholischer Stratohepatitis; atopischen Erkrankungen, Stoffwechselstörungen, neurologischen Störungen, Autoimmunerkrankungen, Unterernährung, chronischem Müdigkeitssyndrom und Autismus.

**14.** Computerimplementiertes Verfahren nach einem der vorstehenden Ansprüche, wobei das Verfahren weiter einen vorherigen Schritt umfasst, in dem mindestens eines der Mikrobiota-Profile hergestellt wird, vorzugsweise wobei der Schritt des Herstellens der Mikrobioata-Profile eine Nukleinsäureanalyse, vorzugsweise eine Nukleinsäuresequenzierung, eine Oligonukleotid-Sondenhybridisierung, Nukleinsäureamplifikation auf Primerbasis; Nachweis auf Basis eines Antikörpers oder eines anderen spezifischen Affinitätsliganden; Proteomanalyse oder Metabolomanalyse umfasst, und vorzugsweise wobei die Probe aus dem GI-Trakt ausgewählt ist aus

(a) Lumeninhalt des GI-Trakts, vorzugsweise Mageninhalt, Darminhalt, Schleim und Kot/Stuhl oder Kombinationen davon,
(b) Teilen der Schleimhaut, der Submukosa, der Muscularis externa, der Adventitia und/oder der Serosa eines Gewebes/Organs des GI-Trakts,
(c) Nukleinsäure, hergestellt aus (a) oder (b), vorzugsweise durch reverse Transkription und/oder Nukleinsäureamplifikation oder
(d) einer mikrobiellen Kultur von (a) oder (b).

**15.** Computer, System oder Einrichtung, die ein Programm tragen, das zur Durchführung des Verfahrens nach einem der vorstehenden Ansprüche ausgelegt ist, vorzugsweise wobei das System oder die Einrichtung weiter zur Durchführung der Mikrobiota-Profilierung oder eines Schritts davon ausgelegt sind.

## Revendications

**1.** Procédé implémenté par ordinateur pour déterminer la probabilité d'une dysbiose du tractus GI chez un sujet, ledit procédé comprenant :

(i) la fourniture d'un ensemble de données de test, dans lequel ledit ensemble de données de test comprend au moins un profil de microbiote, ledit profil de microbiote étant un profil des niveaux relatifs d'une pluralité de micro-organismes ou de groupes de micro-organismes dans un échantillon du tractus GI du sujet et dans lequel chaque niveau de chaque micro-organisme ou groupe de micro-organismes est un élément de profil dudit ensemble de données de test,
(ii) l'application audit ensemble de données de test d'au moins un vecteur de chargement, dans lequel ledit au moins un vecteur de chargement est au moins une variable latente orthogonale déterminée par l'analyse de régression des niveaux de ladite pluralité de micro-organismes ou de groupes de micro-organismes dans des échantillons de tractus GI correspondants provenant d'une pluralité de sujets normaux, ce qui permet de produire un premier ensemble de données projeté,
(iii) la fourniture dudit premier ensemble de données projeté,
(iv) à partir dudit premier ensemble de données projeté, le calcul du résidu Q du profil de microbiote et la comparaison du résidu Q du profil de microbiote avec une valeur de résidu Q de seuil normobiotique à dysbiotique déterminée à partir de l'analyse correspondante de ladite pluralité de micro-organismes ou de groupes de micro-organismes dans des échantillons de tractus GI correspondants provenant d'une pluralité de sujets normaux et/ou de sujets présentant une dysbiose,

(v) à partir dudit premier ensemble de données projeté, le calcul du $T^2$ de Hotelling pour le profil de microbiote à partir de la variance expliquée par les variables latentes orthogonales déterminées par l'analyse de régression des niveaux de ladite pluralité de micro-organismes ou de groupes de micro-organismes dans des échantillons de tractus GI correspondants provenant d'une pluralité de sujets normaux de l'étape (ii) et la comparaison dudit $T^2$ de Hotelling pour le profil de microbiote avec une valeur de $T^2$ de Hotelling de seuil normobiotique à dysbiotique déterminée à partir de l'analyse correspondante de ladite pluralité de micro-organismes ou de groupes de micro-organismes dans des échantillons de tractus GI correspondants provenant d'une pluralité de sujets normaux et/ou d'une pluralité de sujets présentant une dysbiose,

dans lequel l'étape (v) peut être réalisée avant ou après ou simultanément avec l'étape (iv) et dans lequel un profil de microbiote avec un résidu Q ou un $T^2$ de Hotelling au-dessus desdits seuils respectifs indique une probabilité de dysbiose.

2.  Procédé implémenté par ordinateur selon la revendication 1, dans lequel ledit ensemble de données de test comprend une pluralité de profils de microbiote et ledit ensemble de données de test est agencé dans une matrice.

3.  Procédé implémenté par ordinateur selon la revendication 1 ou la revendication 2, dans lequel lesdites variables latentes orthogonales sont déterminées par la transformation orthogonale en composants principaux des niveaux de ladite pluralité de micro-organismes ou de groupes de micro-organismes dans des échantillons de tractus GI correspondants provenant d'une pluralité de sujets normaux, de préférence dans lequel la transformation orthogonale en composants principaux se fait par au moins l'une d'une analyse partielle de régression des moindres carrés, d'une analyse de composant principal, d'une analyse canonique des corrélations, d'une analyse de redondance, d'une analyse des correspondances et d'une analyse canonique des correspondances.

4.  Procédé implémenté par ordinateur selon une quelconque revendication précédente, dans lequel au moins 2 vecteurs de chargement, de préférence au moins 3, 5, 7, 9, 11, 13, 15, 17, 19 ou 20 vecteurs de chargement et/ou pas plus de 50 vecteurs de chargement, de préférence pas plus de 40, 30, 25, 20 ou 15 vecteurs de chargement sont appliqués.

5.  Procédé implémenté par ordinateur selon une quelconque revendication précédente, dans lequel le vecteur de chargement est appliqué sous la forme d'une matrice de projection.

6.  Procédé implémenté par ordinateur selon une quelconque revendication précédente, dans lequel lesdits profils de microbiote sont quantitatifs ou semi-quantitatifs et dans lequel ledit procédé fournit une mesure quantitative ou semi-quantitative de l'étendue de la dysbiose.

7.  Procédé implémenté par ordinateur pour quantifier une dysbiose, ledit procédé comprenant la réalisation du procédé selon la revendication 6, dans lequel lesdites comparaisons avec des seuils normobiotiques à dysbiotiques comprennent ensemble la combinaison du résidu Q de l'étape (iv) et du $T^2$ de Hotelling de l'étape (v) en une métrique unique pour la dysbiose, de préférence dans lequel la distance euclidienne depuis l'origine à la fois pour le résidu Q de l'étape (iv) et le $T^2$ de Hotelling de l'étape (v) est calculée et, de préférence, dans lequel la distance euclidienne depuis l'origine à la fois pour le résidu Q et le $T^2$ de Hotelling est calculée avec la formule I :

$$r = \sqrt{\{T^2\}^2 + Qres^2}$$

8.  Procédé implémenté par ordinateur selon la revendication 7, dans lequel la combinaison de le résidu Q de l'étape (iv) et le $T^2$ de Hotelling de l'étape (v) en une métrique unique pour la dysbiose comprend une mise à l'échelle dudit résidu Q de l'étape (iv) et du $T^2$ de Hotelling de l'étape (v) pour avoir pour résultat des valeurs de grandeur similaire.

9.  Procédé implémenté par ordinateur selon la revendication 7 ou la revendication 8, dans lequel ladite métrique unique est tracée sur une échelle numérique finie avec une séparation de classe normobiotique à dysbiotique à un point prédéterminé sur ladite échelle numérique finie qui représente, ou est, une combinaison des seuils de classe normobiotique à dysbiotique des étapes (iv) et (v), mis à l'échelle de manière similaire si une mise à l'échelle a été appliquée.

10. Procédé implémenté par ordinateur selon la revendication 7 ou la revendication 8, dans lequel ladite métrique unique est tracée sur une échelle numérique finie avec une séparation de classe normobiotique à dysbiotique à un point prédéterminé sur ladite échelle numérique finie et dans lequel

(a) pour un échantillon de test présentant au moins l'un du résidu Q de l'étape (iv) ou du $T^2$ de Hotelling de l'étape (v) au-dessus des valeurs de seuil de classe normobiotique à dysbiotique des étapes (iv) et (v), respectivement, ledit point de séparation de classe correspond à celui de l'une ou de l'autre de la valeur de seuil de classe normobiotique à dysbiotique des étapes (iv) ou (v) dépassée, mises à l'échelle de manière similaire si une mise à l'échelle a été appliquée et

(b) pour un échantillon de test dans lequel ni le résidu Q de l'étape (iv) ni le $T^2$ de Hotelling de l'étape (v) ne sont en dessous des valeurs de seuil normobiotique à dysbiotique des étapes (iv) et (v), respectivement, ledit point de séparation de classe correspond à la somme des seuils de classe normobiotique à dysbiotique des étapes (iv) et (v), mis à l'échelle de manière similaire si une mise à l'échelle a été appliquée.

11. Procédé implémenté par ordinateur selon l'une quelconque des revendications 7 à 10, dans lequel des pondérations sont appliquées sur le résidu Q de l'étape (iv) et le $T^2$ de Hotelling de l'étape (v) pendant la seconde étape de combinaison et dans lequel lesdites pondérations réduisent à un minimum les effets d'une variation technique.

12. Procédé implémenté par ordinateur pour diagnostiquer, surveiller et/ou caractériser des maladies et des états associés à des perturbations dans le microbiote du tractus GI ou à l'évaluation du risque de développer une maladie ou un état qui est associé à une perturbation du profil de microbiote du tractus GI, ledit procédé comprenant la réalisation d'un procédé tel que défini dans l'une quelconque des revendications 1 à 11, dans lequel l'indication de la probabilité d'une dysbiose ou de l'étendue de la dysbiose indique la présence, ou l'absence, le risque de développer, le progrès de ou les caractéristiques de ladite maladie ou dudit état associé à des perturbations dans le microbiote du tractus GI.

13. Procédé implémenté par ordinateur selon la revendication 12, dans lequel ladite maladie ou ledit état associé à une perturbation dans le microbiote du tractus GI est sélectionné parmi des troubles fonctionnels du tractus GI, le syndrome de prolifération bactérienne dans l'intestin grêle, des cancers du tractus GI, le cancer du sein, la spondylarthrite ankylosante ; la stéatohépatite non alcoolique ; des maladies atopiques, des troubles métaboliques, des troubles neurologiques, des maladies auto-immunes, la malnutrition, le syndrome de fatigue chronique et l'autisme.

14. Procédé implémenté par ordinateur selon une quelconque revendication précédente, dans lequel ledit procédé comprend en outre une précédente étape au cours de laquelle au moins un des profils de microbiote est préparé, de préférence dans lequel ladite étape de préparation desdits profils de microbiote comprend une analyse d'acide nucléique, de préférence le séquençage des acides nucléiques, l'hybridation de sonde oligonucléotidique, une amplification des acides nucléiques à base d'amorce; une détection basée sur un anticorps ou un autre ligand à affinité spécifique ; une analyse protéomique ou une analyse métabolomique et, de préférence, dans lequel l'échantillon provenant du tractus GI est sélectionné parmi

(a) le contenu luminal du tractus GI, de préférence le contenu de l'estomac, le contenu intestinal, le mucus et les fèces/matières fécales ou des combinaisons de ceux-ci,
(b) des parties de la muqueuse, de la sous-muqueuse, de la tunique musculaire externe, de l'adventice et/ou de la séreuse d'un tissu/organe du tractus GI,
(c) un acide nucléique préparé à partir de (a) ou de (b), de préférence par une transcription inverse et/ou une amplification des acides nucléiques ou
(d) une culture microbienne de (a) ou de (b).

15. Ordinateur, système ou appareil comportant un programme conçu pour réaliser le procédé selon une quelconque revendication précédente, de préférence dans lequel ledit système ou appareil est en outre conçu pour réaliser un profilage du microbiote ou une étape de celui-ci.

Figure 1

Figure 2

Figure 3

Figure 4

Figure 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012080754 A
- WO 2011043654 A
- WO 9851693 A
- WO 0153525 A

### Non-patent literature cited in the description

- **VEBO HC ; SEKELJA M ; NESTESTOG R et al.** Temporal development of the infant gut microbiota in immunoglobulin E-sensitized and nonsensitized children determined by the GA-map infant array. *Clin Vaccine Immunol,* 2011, vol. 18, 1326-1335